(19) Europäisches Patentamt
European Patent Office
Office européen des brevets

(11) Publication number: 0 104 611 A2

# (12) EUROPEAN PATENT APPLICATION

(21) Application number: 83109436.2

(22) Date of filing: 22.09.83

(51) Int. Cl.³: C 07 D 417/12
C 07 D 285/00, C 07 D 417/14
A 61 K 31/54

(30) Priority: 28.09.82 GB 8227614

(43) Date of publication of application:
04.04.84 Bulletin 84/14

(84) Designated Contracting States:
AT BE CH DE FR GB IT LI LU NL SE

(71) Applicant: HOECHST U.K. LIMITED
Hoechst House Salisbury Road
Hounslow Middlesex TW4 6JH(GB)

(72) Inventor: Ross, Barry Clive
5 Upton Close
Luton Bedfordshire(GB)

(72) Inventor: Michael, Jeffrey Daniel
5 Wood Street Woburn Sands
Milton Keynes Buckinghamshire(GB)

(72) Inventor: Cousins, Simon John
32 Durrans Court Fenny Stratford
Milton Keynes Buckinghamshire(GB)

(74) Representative: Meyer-Dulheuer, Karl-Hermann, Dr. et al,
HOECHST Aktiengesellschaft Zentrale Patentabteilung
Postfach 80 03 20
D-6230 Frankfurt/Main 80(DE)

## (54) Thiatriazine derivatives.

(57) New thiatriazine derivatives of the general formula I

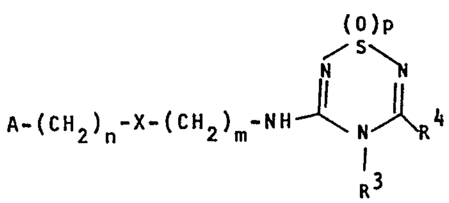

(I)

are described as well as a process for their manufacture. The new compounds exhibit histamine H-2 antagonist activity and may be used to inhibit gastric acid secretion and to treat gastric and peptic ulcers.

EP 0 104 611 A2

The present invention relates to thiatriazine derivatives which have histamine H-2 antagonist activity. The invention also relates to processes for the preparation of these derivatives, to pharmaceutical preparations comprising them, and to their use.

Histamine is one of a number of naturally occurring physiologically active substances which are thought to interact with specific receptors. In the case of histamine, there are at least two types: one is called H-1 receptor (Ash and Schild, Brit. J. Pharmac. 1966, 27 427), and the other is called the H-2 receptor (Black et al Nature 1972, 236, 385). The action of histamine at the H-1 receptors, for example, stimulation of bronchial and gastro-intestinal smooth muscle, is blocked by the compounds generally known as "antihistamines", but which are now also called histamine H-1 antagonists, for example, mepyramine. The action of histamine at the H-2 receptors, for example, stimulation of gastric acid secretion and heart rate, is not blocked by mepyramine, but is blocked by other compounds, for example, burimamide and cimetidine.

Histamine H-2 antagonists may be used to treat those conditions resulting from stimulation by histamine of H-2 receptors, either alone, for example, in inhibiting gastric acid secretion and thus treating its sequelae, for example, gastric and peptic ulcers; or together with H-1 antagonists, for example, in allergic and certain inflammatory conditions.

The present invention provides compounds of the general formula I, which are histamine H-2 antagonists:

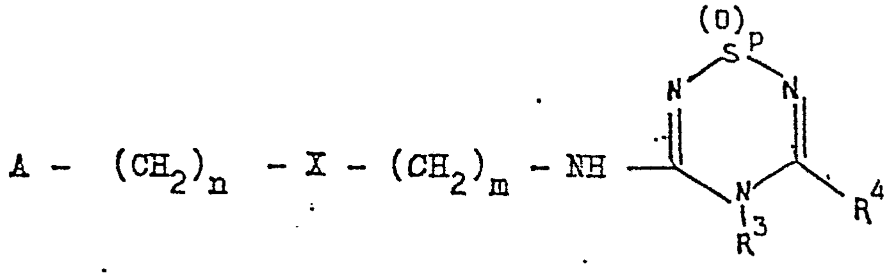

(I)

in which formula

A represents a phenyl, imidazolyl, thiazolyl, furyl, thienyl, or pyridyl radical; which radical may contain one or two substitutents, the first being selected from lower alkyl groups, and the second from lower alkyl, guanidino, and $-CH_2NR^1R^2$ groups, $R^1$ and $R^2$, which may be the same or different, each representing a hydrogen atom or a $(C_1-C_6)$alkyl group, or together with the nitrogen atom to which they are attached, may form a pyrrolidine, piperidine, morpholine, or N-methylpiperazine ring;

X represents -O-, -S-, or $-CH_2$;

n represents 0 or 1;

m represents 2 or 3;

p represents 1 or 2;

$R^3$ represents a hydrogen atom or a $(C_1-C_6)$alkyl, $(C_2-C_6)$-alkenyl, $(C_3-C_6)$alkynyl, phenyl, phenyl(lower)alkyl, carboxylic acyl$(C_1-C_6)$, phenyl(lower)acyl, nitrile, or $-N(alkyl)_2$ group;

$R^4$ represents a hydrogen atom, a $(C_1-C_6)$alkyl, $(C_2-C_6)$-alkenyl, $(C_3-C_6)$alkynyl, phenyl or phenyl(lower)alkyl group, an -O(lower)alkyl or -Oaryl group, a nitrile group, or an $-NR^5R^6$ group, in which $R^5$ and $R^6$, which may be the same or different, each represents a hydrogen atom, a $(C_1-C_6)$alkyl, $(C_2-C_6)$-alkenyl, $(C_3-C_6)$alkynyl, $(C_3-C_7)$cycloalkyl, phenyl or phenyl(lower)alkyl group, a $-(CH_2)_m-X-(CH_2)_n-A$ group, or an $-NH_2$ or nitrile group, (m, n, X and A being as defined above), or together with the nitrogen atom to which they are attached may form a 5 or 6 membered ring optionally containing a second nitrogen atom or an oxygen atom, or

$R^3$ and $R^4$ together with the atoms to which they are attached may form a 5 or 6 membered ring, containing one or more further heteroatoms selected from oxygen, nitrogen and sulphur atoms.

This invention also provides salts of a compound of formula I, especially physiologically tolerable salts.

Alkyl, alkenyl, alkynyl, and acyl groups in the present specification may be branched or unbranched and may be unsubstituted or substituted, for example, by one or more groups selected from hydroxyl groups; $-OR^7$ groups in which $R^7$ represents a $(C_1-C_6)$alkyl group, $(C_3-C_7)$cycloalkyl or a phenyl group; $-NR^8R^9$ groups in which $R^8$ and $R^9$, which may be the same or different, each represents a hydrogen atom, a lower alkyl group, an aryl group or a lower acyl group, and may together with the nitrogen atom to which they are attached form a 5 or 6 membered ring; $-COOR^{10}$ groups in which $R^{10}$ represents a hydrogen atom, an alkali metal atom, for example, Na or K, or a lower alkyl group; $-CONR^8R^9$ in which $R^8$ and $R^9$ are as defined above; lower alkylsulphonyl and arylsulphonyl groups; cyano groups; and phenyl groups which may be substituted by one or two substituents, which may be the same or different selected from a lower alkyl, lower alkoxy, methylenedioxy, phenoxy, halogen, dimethylaminomethyl, trifluoromethyl, nitro, cyano, sulphonic acid, sulphonamide, amino, mono- lower alkyl, amino di-lower alkyl amino groups; aromatic and non-aromatic heterocyclic groups having 5 to 8 ring members and one or two hetero atoms selected from oxygen, sulphur and nitrogen, and optionally having a lower alkyl substituent on a ring nitrogen atom, for example, furyl, tetrahydrofuryl, thienyl, pyridyl, dihydropyranyl, pyrrolidinyl, N-lower alkyl, pyrrolidinyl and piperidyl substituent.

A phenyl group other than a phenyl group A (which is defined above) may be unsubstituted or substituted as defined immediately above for phenyl substituents of aliphatic and acyl groups.

In the present specification the term "lower" is used to denote a group having up to 4 carbon atoms. The term "halogen" denotes chlorine, bromine, iodine and fluorine. The term "aryl", unless otherwise described, denotes aromatics having 6 to 12 carbon atoms, such as phenyl, naphthyl, bisphenylyl, particularly phenyl;

aralkyl means aralkyls having 7 to 13 C atoms, such as benzyl, phenethyl.

It will be appreciated by a worker skilled in the art that a combination of substituents that are incompatible for steric reasons or because of potential inter-reactions should not be chosen. The worker will also use his normal discretion in the number of substituents chosen.

The present invention also provides a process for the production of a compound of the general formula I, which comprises

a) reacting a 1,2,4,6-thiatriazine of the general formula II

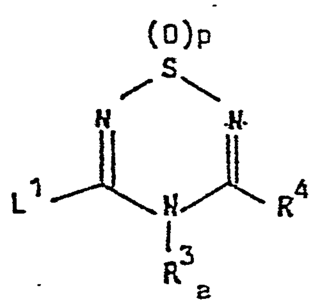

II

in which $L^1$ represents a halogen atom, an alkoxy or aryloxy group, an alkylthio or arylthio group, or an alkylsulphonyl or arylsulphonyl group, p and $R^4$ are as defined above, and $R^3_a$ us as defined above for $R^3$ except that $R^3_a$ may not represent a hydrogen atom, with a compound of the general formula III

$$A-(CH_2)_n-X-(CH_2)_m-NH_2 \qquad III$$

in which A, X, m and n are as defined for formula I, or

b) reacting a 1,2,4,6-thiatriazine of the general formula IV

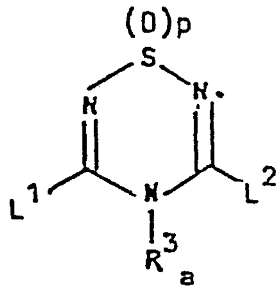

IV

in which $L^1$ and $L^2$, which may be the same or different, each represents a halogen atom, an alkoxy or aryloxy group, an alkylthio or arylthio group, or an alkylsulphonyl or arylsulphonyl group, and p and $R^3_a$ are as defined above, with a compound of formula III as defined above, and reacting the resulting compound of formula V

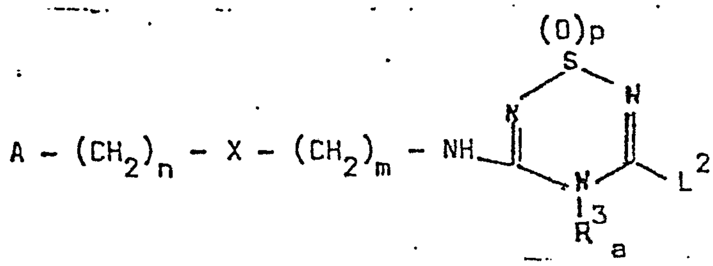

V

with an amino compound $HNR^5_aR^6_a$ in which $R^5_a$ and $R^6_a$ are as defined for $R^5$ and $R^6$ in formula I and, in addition, either or both of $R^5_a$ and $R^6_a$ may represent a trialkylsilyl group, for example, a $-(CH_3)_3Si$ group; or

c) reacting a 1,2,4,6-thiatriazine of the general formula II as defined above with an amino thiol of formula VI

$$HX_a - (CH_2)_m - NH_2 \qquad VI$$

in which $X_a$ is -O- or -S-, and m is as defined for formula I, and reacting the resulting a compound of formula VII

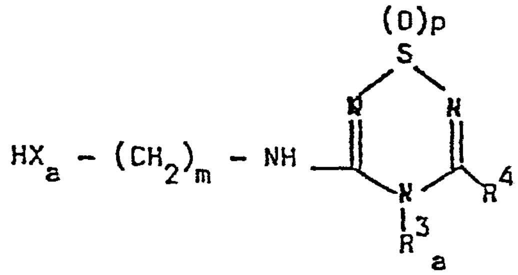

VII

in which $R^3_a$, $R^4$, p and m are as defined above, with a compound of the general formula VIII

$$A - (CH_2)_{n^1} - L^3 \qquad VIII$$

in which A is as defined for formula I, $n^1$ represents 1, and $L^3$ is a suitable leaving group, for example, a halogen atom, a hydroxy group, an alkoxy group, or a sulphonate ester, and, if desired, carrying out any one or more of the following reactings in any desired order:-

(i) converting a group $R^3_a$ into a hydrogen atom,

(ii) converting a group $R^3$ and/or a group $R^4$ into another group $R^3$ and/or $R^4$, respectively,

(iii) converting an acid addition salt of formula I into the corresponding free base or converting a free base into an acid addition salt,

(iv) oxidising a compound or formula I in which p represents 1 to give the corresponding compound in which p represents 2.

In addition to serving as a substituent in the final product, $R^3$ may also act as a protecting group in the above chemical transformations and may subsequently be removed, for example, by hydrogenolysis or by acid or base catalysed hydrolysis to provide a compound of the general formula I in which the thiatriazine ring carries a hydrogen substituent. Protecting groups which come within the definition of

$R^3$ given in formula I are t-butyl, lower alkyl carbonate, benzyl and benzoyl groups, for example.

Oxidation may be carried out by a know method, for example, as described below. Interconversions of substitutents may also be carried out by known methods, for example, the hydrolysis of a cyano group to an acid or amide.

Compounds II and III are generally reacted in a solvent or diluent, preferably an alcohol, DMF, or DMSO, at a temperature within the range of from, for example, from 0 to 100°C, generally from 0 to 60°C. The compound of formula III should be reacted in the form of the free base, as shown. If it is initially present in the form of an acid addition salt, for example, as the hydrochloride or hydrobromide, this should be converted into the free base during or, preferably, after reaction with compound II. Conversion is carried out with a base, for example, triethylamine, sodium hydroxide or potassium hydroxide.

Compound IV is reacted with compound III under conditions similar to those described above, except that generally lower reaction temperatures are preferred in order that the leaving groups $L^1$ and $L^2$ may be displaced selectively.

The reaction of compound V with an amino compound $HNR^5{}_aR^6{}_a$ is generally carried out in an alcoholic solvent at a temperature from 0 to 60°C. The reaction in all cases is faster when p= 2 than when p= 1.

A compound of formula I may be converted into its salt form in the usual manner by reaction with an acid. Examples of physiologically tolerable acid addition salts are those with hydrobromic, hydrochloric, sulphuric, acetic, malonic, maleic, fumaric, succinic, citric, tartaric and isethionic acids.

A compound of formula I in the form of an acid addition salt may be converted into the free base form by reaction with a base in the usual manner.

A number of compounds of the type represented by the general formula III are known, see, for example, GB Specification 2,001,624 A; U.S. Patent 3,950,333; U.S. Patent 4,128,658; and Belgian Patents 867,106 and 875,846.

A few examples of the required thiatriazine intermediates represented by the general formulae II, IV and VI are disclosed in the chemical literature, see for example, German specification DE 2,026,625 and DE 2,943,703; U.S. patent 4,013,447; Heterocycles 12, 1199 (1979); and Chem. Ber. 109 2107 (1976).

However, many of the specific intermediates required for the present invention have not previously been described and form part of the present invention.

Some thiatriazines of the general formula II may be prepared as shown in Scheme I.

Scheme I

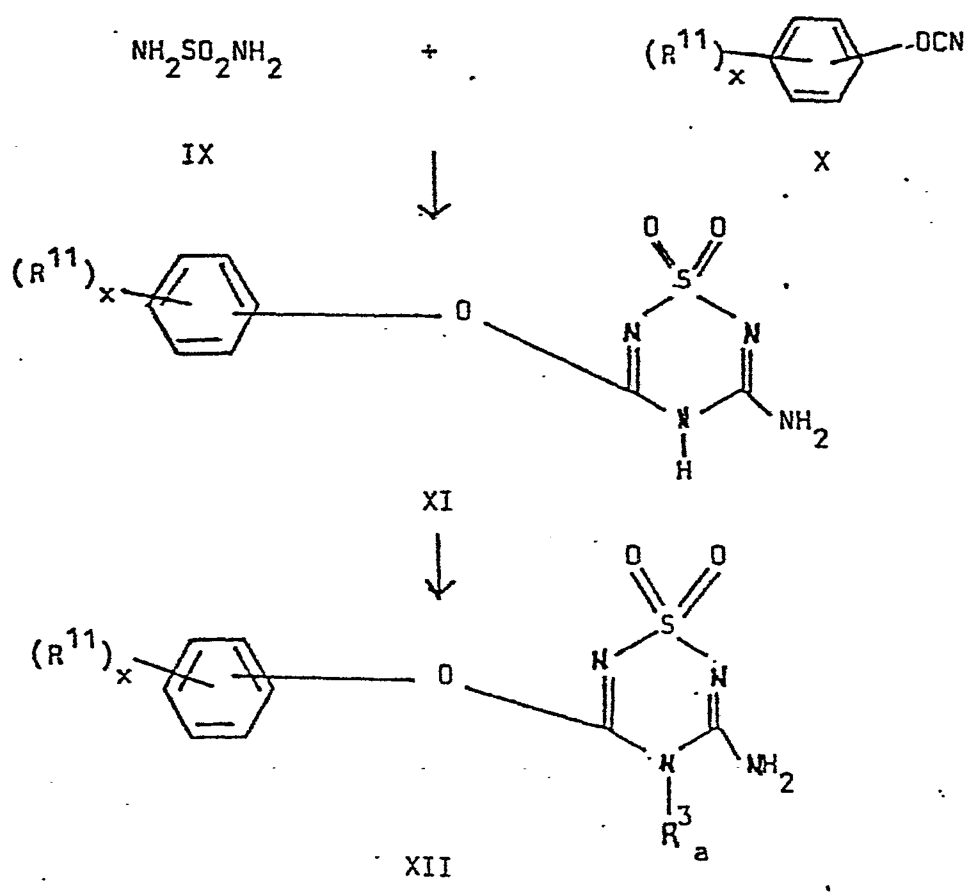

IX

X

XI

XII

$R^{11}$ represents an alkyl, dialkylamino, acylamino, nitro, halogen, aryl, nitrile, alkoxy, aryloxy or acyloxy group, and x represents 1 or 2. Preferably one group $R^{11}$ is present, ortho or, preferably, para to the -OCN group.

Sulphamide IX may be reacted with an aryl cyanate of formula X in the presence of a base, for example, $Na_2CO_3$, according to the method described in DE 2,026,625 to give a thiatriazine of formula XI. This compound may be alkylated on the nitrogen atom in the 4-position using an alkylating agent, for example, an alkyl halide or alkyl sulphate, for example, methyl iodide, benzyl bromide or dimethyl sulphate.

The alkylation of the 4-amino group may be carried out in a known manner, for example, in the presence of a base, for example, sodium methoxide or di-isopropylethylamine, in a solvent, for example, methanol or acetonitrile, and generally at a temperature within the range of 20 to 100°C.

Certain thiatriazines of the general formula IV may be prepared as shown in Scheme II or Scheme III.

Scheme II

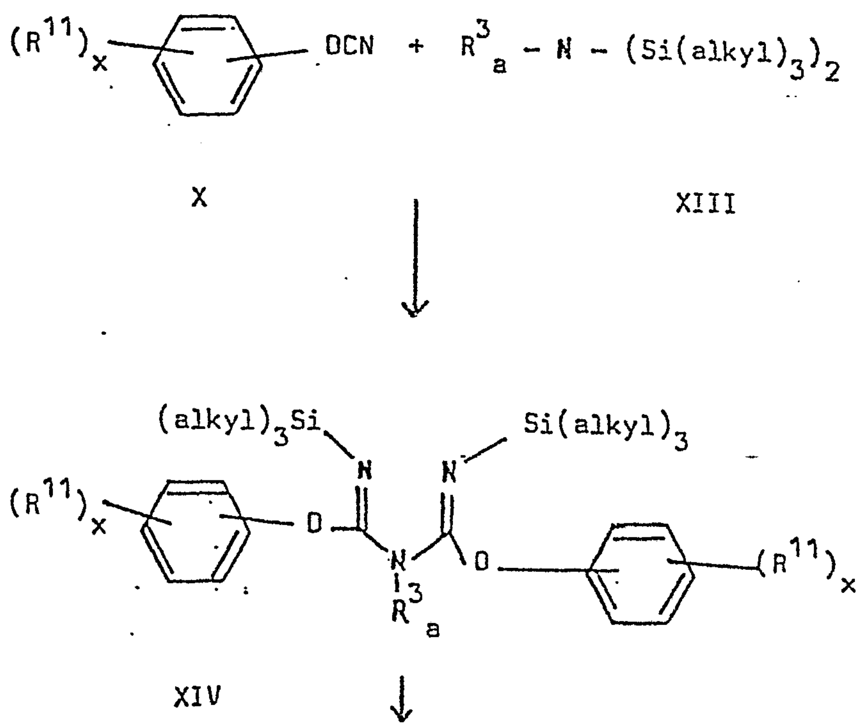

X

XIII

XIV

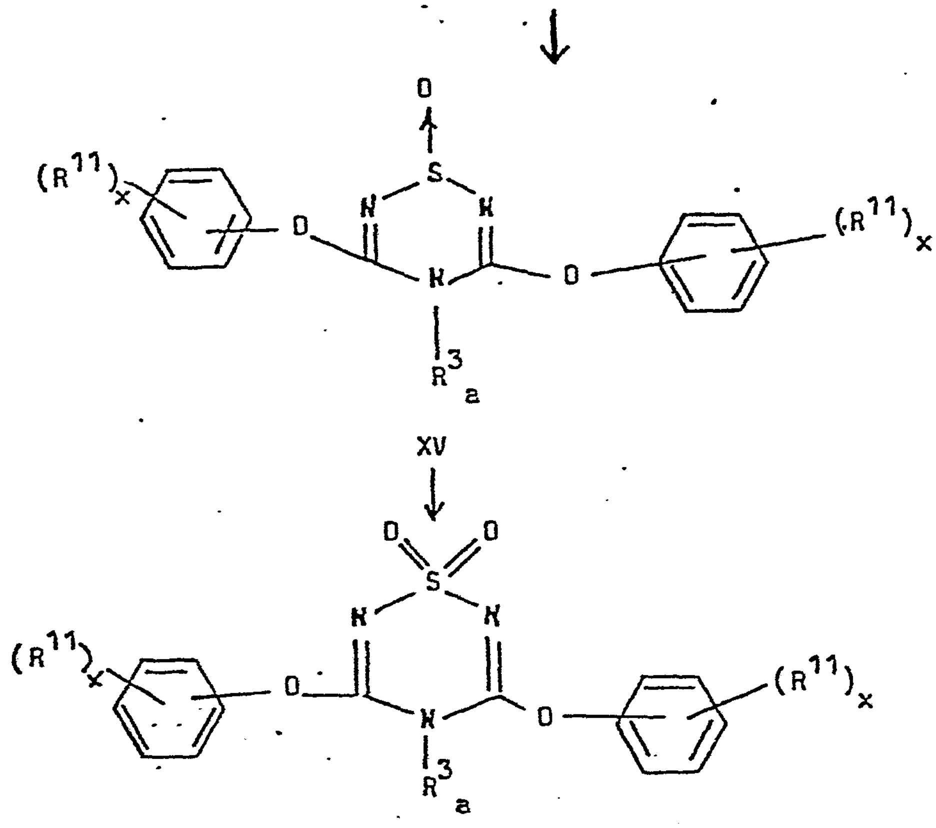

XV

XVI

In Scheme II, an aryl cyanate of formula X in which $R^{11}$ and x are as defined above is reacted with a substituted bis-trialkylsilylamine of formula XIII in which $R^3_a$ is as defined above to give a compound of formula XIV. The trialkyl group is preferably a trimethyl group. This reaction may be carried out as described in Chem. Ber. 101 3185 (1968). Cyclisation of compound XIV to give the thiatriazine of formula XV may be carried out by reaction of compound XIV with thionyl chloride, thionylaniline or thionyldiimidazole. A compound XV may be used diretly in a condensation reaction with a compound of formula III to give a compound of formula I, or may first be oxidised to the corresponding 1,1-dioxide XVI, using an oxidising agent, for example, a peracid, for example, m-chloroperbenzoic acid, hydrogen peroxide, an alkyl hydroperoxide, for example, t-butyl hydroperoxide, or a permanganate, for example potassium permanganate.

Scheme III

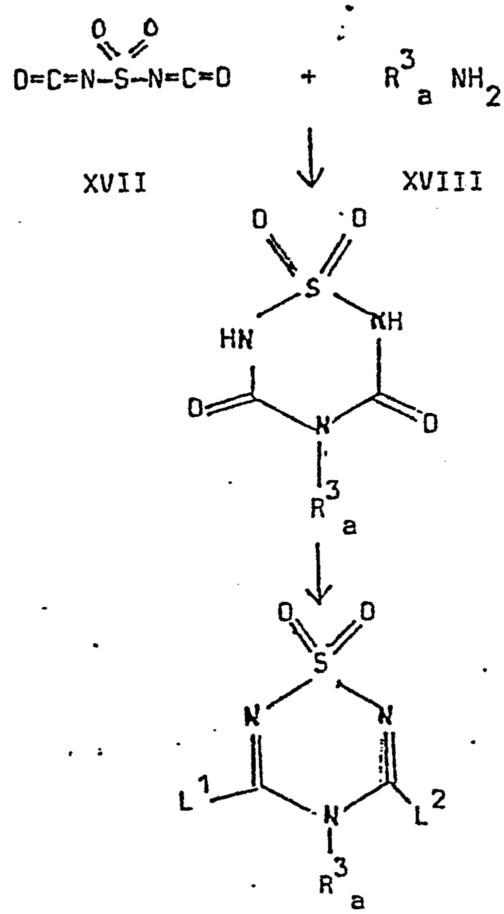

XVII XVIII

XIX

XX

Sulphonyl di-isocyanate XVII may be reacted with a primary amine of formula XVIII in which $R^3_a$ is as defined above according to DE-OS 2,337,867, to provide the thiatriazine of formula XIX. Conversion of this compound to the thiatriazine XX having leaving groups $L^1$ and $L^2$ at the 3 and 5 positions (ie a compound of formula IV in which p= 2) may be accomplished by a variety of methods, for example, by treatment with $PCl_5$ or $COCl_2$/DMF in a solvent, for example, $POCl_3$, $CCl_4$ or $ClCH_2CH_2Cl$, zo give $L^1 = L^2 = Cl$, or by treatment with phosphorus pentasulphide or p-methoxyphenylthionophosphine sulphide dimer with subsequent alkylation to give $L^1 = L^2$ = alkylthio.

0104611

It will be appreciated by those skilled in the art that it is possible to convert a thiatriazine intermediate of formula IV into a thiatriazine intermediate of formula II by, for example, reaction with a primary or secondary amine $NHR^5_aR^6_a$.

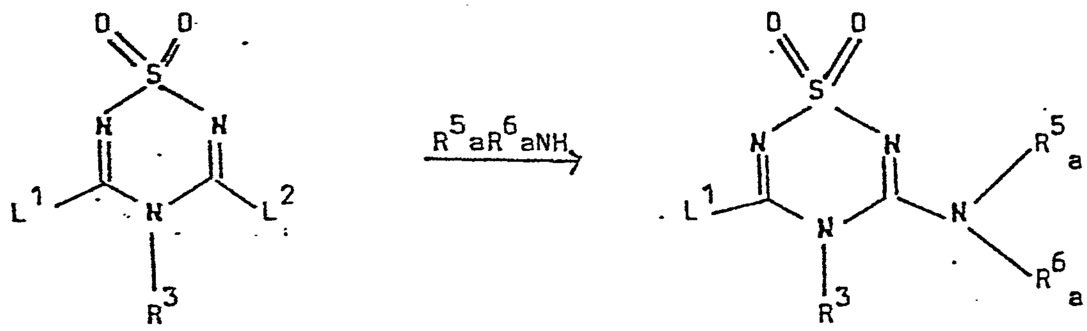


IV

II

Certain thiatriazines of general formula II may be prepared as shown in Scheme IV.

Scheme IV

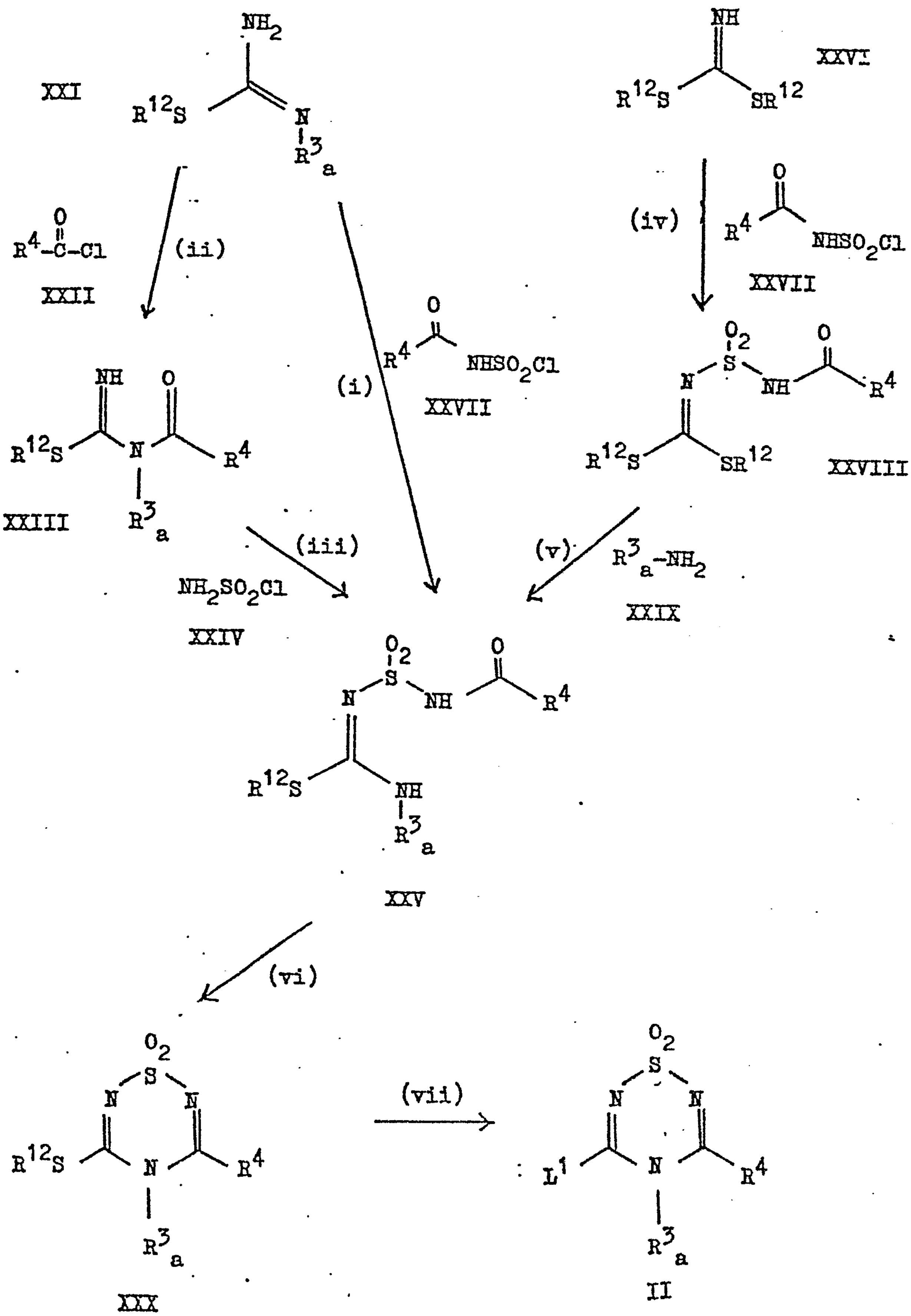


XXI

XXII

XXIII

XXIV

XXV

XXVI

XXVII

XXVIII

XXIX

XXX

II

In the above reaction scheme, $R^3_a$, $R^4$ and $L^1$ are as defined above, and $R^{12}$ represents an alkyl group, for example an alkyl group having up to 4 carbon atoms, especially a methyl group; an aryl group, especially a phenyl group; or an aralkyl group, especially a benzyl group. In compound XXVI, the two groups $R^{12}$ may be the same or different. In compound XXI, $R^{12}$ is for example, a methyl group, and in compound XXVI, for example, one of the two groups $R^{12}$ is a methyl group and the other is a phenyl group, or both are methyl groups, or both are phenyl groups.

Acylaminosulphonylchlorides of formula XXVII are known, see for example, R. Graf, German Specifications 931,225 and 931,467. Dithioiminocarbonates of formula XXVI are also known, see for example, Z. Chem. 8, 459 - 460 (1968). (The term "known" is used herein to mean in actual use in the art or described in the literature of the art.)

An N-substituted isothiourea XXI may be reacted with an acylaminosulphonyl chloride XXVII in the presence of a base, for example, triethylamine or diisopropylethylamine, in an aprotic solvent, for example, dichloromethane, tetrahydrofuran or acetonitrile, generally at a temperature within the range of from -20 to +30°C, to give the key intermediate of formula XXV (reaction i).

A compound of formula XXV may also be prepared from an isothiourea XXI by another route (reactions ii and iii).

First, the isothiourea XXI reacted with a carboxylic acid chloride XXII, in the presence of a base, for example, triethylamine or diisopropylethylamine, in an aprotic solvent, for example, dichloromethane, tetrahydrofuran or acetonitrile, generally at temperature within the range of from -50 to 0°C, to give a mixture of isomers from which the predominant one, compound XXIII, can be separated by conventional means, for example, by fractional crystallisation or by chromatography. Compound XXIII is then reacted with aminosulphonyl chloride XXIV under conditions analogous to those described above for reaction (i). Migration of the acyl group results in the formation of compound XXV.

Compound XXV may be prepared by a third method (reactions iv and v), which comprises reacting a dithioiminocarbonate XXVI with an acylaminosulphonyl chloride XXVII under conditions analogous to those described above for reaction (i) and then displacing an $-SR^{12}$ group from the resulting compound XXVIII with primary amine of formula XXIX.

Heating a compound of formula XXV in an aprotic solvent, for example, tetrahydrofuran, dioxane, toluene, xylene or ethylene glycol dimethyl ether, generally at a temperature within the range of from 80 to 160°C, affords a thiatriazine of formula XXX.

In some compounds of formula XXX,the group $R^{12}S-$ is a suitable leaving group $L^1$, ie such compounds fall within the general formula II, but in other compounds of formula XXX it is necessary or preferable to convert a goup $R^{12}S-$ into a leaving group $L^1$ that is more readily displaced by a nucleophile. Such a conversion may be carried out by known methods, for example, by oxidising the sulphide group $R^{12}S-$ to the corresponding sulphoxide group using, for example, a peracid, for example, m-chloroperbenzoic acid, hydrogen peroxide, or a periodate, perborate or permanganate salt; or by replacing the sulphide group $R^{12}S-$ by a halogen atom, for example, by treating compound XXX with excess chlorine in an inert solvent, for example, chloroform, tetrahydrofuran or ethyl acetate, generally at a temperature within the range of from 20 to 100°C, and preferably in the presence of a catalyst, for example, zinc chloride.

It will be appreciated by those skilled in the art that compounds of the general formula I may exist as one or more tautomeric isomers, especially those structures where $R^3$ represents a hydrogen atom and $R^4$ represents a group $-NR^5R^6$ in which $R^5$ or $R^6$ represents a hydrogen atom, for example, as shown below:

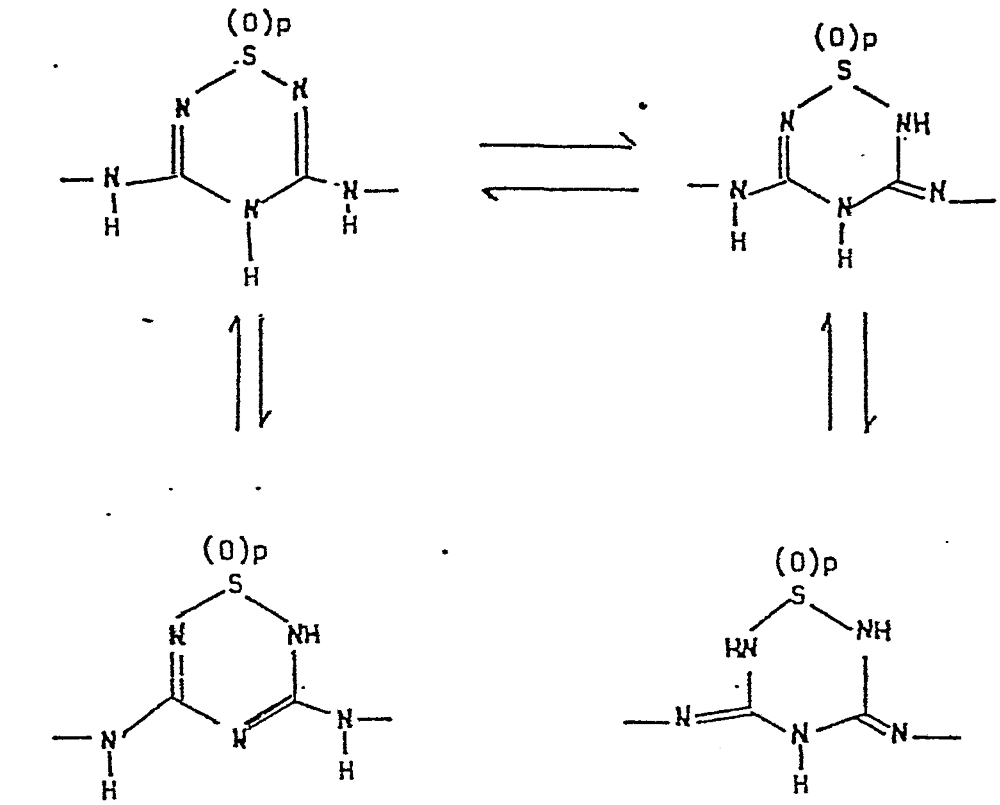

This tautomerism may also occur when the thiatriazine ring is substituted by carbon substituents:

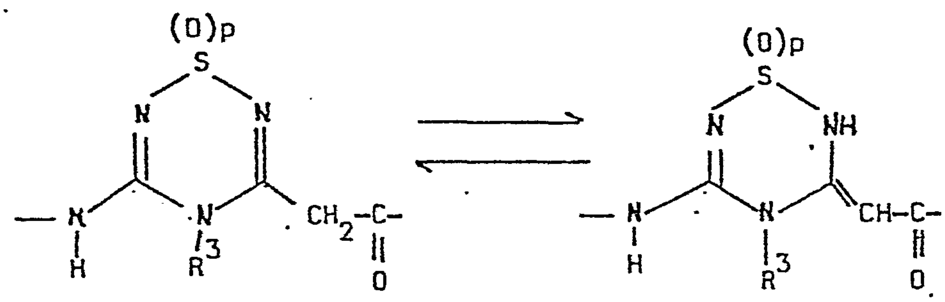

All possible tautomeric forms of formula I are part of the present invention.

It will also be appreciated that when p represents 1, the sulphur-oxygen bond in formula I can exist in two stereoisomeric configurations: R and S. All stereoisomers of formula I are also part of the present invention.

Any reference to a compound of formula I herein includes all possible tautomeric and stereoisomeric forms of that compound. Moreover, all possible tautomeric and stereoisomeric forms of any other compound mentioned in the present specification are similarly included within the general references to that compound.

As indicated above, the compounds of the present invention have histamine H-2 antagonist activity. Histamine $H_2$-antagonists have been shown to inhibit gastric acid secretion in a variety of test animals and in man. These compounds are also capable of blocking the gastric secretion stimulated by exogenously administered histamine or pentagastrin; see Brimblecombe et al., J. Int. Med. Res. 3, 86 (1975).

Compounds of this invention were tested for antisecretory activity in the perfused rat stomach. The method of Gosh and Schild, Brit. J. Pharmacol. 13, 54 (1958) as modified by Lawrence and Smith, Europ. J. Pharmacol. 25, 389 (1974) was used to prepare the test animals and acid secretion was stimulated by infusing either histamine or pentagastrin at a constant rate.

After the secretion plateau was reached, the test compound was administered i.v. and the amount of comound required to inhibit the stimulated secretion by 50 % was determined.

The $ID_{50}$ values for some of the compounds of this invention are given in the following Table. Two to four rats were used for each determination.

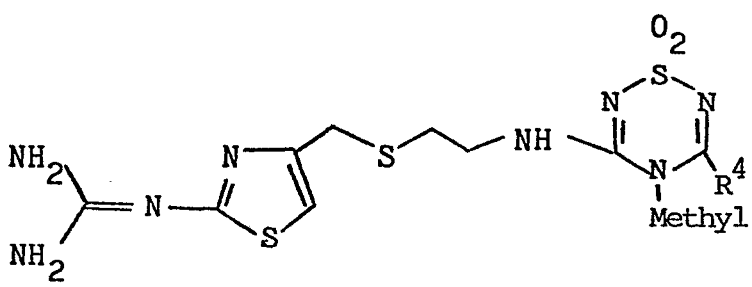

| $R^4$= | $ID_{50}$ (μmoles/kg) Rat perfused stomach i.v. |
|---|---|
| Cimetidine | 10.8 |
| 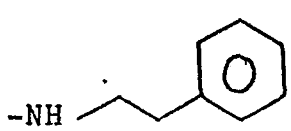 | 0.083 |
| 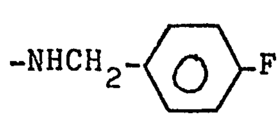 | 0.065 |

| | |
|---|---|
| 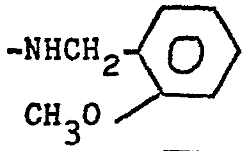 | 0.10 |
| 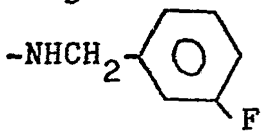 | 0.10 |
| 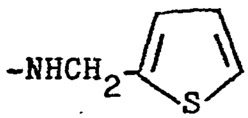 | 0.022 |
| $-NHCN$ | 0.34 |
| $-NH_2$ | 0.15 |
| $-N(CH_3)_2$ | 0.095 |
| 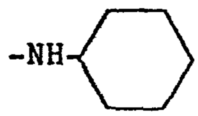 | 0.12 |
| $-NHCH_2C{\equiv}CH$ | 0.12 |
| $-NH-CH(CH_3)_2$ | 0.28 |

Accordingly, the present invention provides a pharmaceutical preparation which comprises a compound of the general formula I or a physiologically tolerable salt thereof as active ingredient, in admixture or conjunction with a pharmaceutically suitable carrier. The preparation may be in a form suitable for enteral or parenteral adminstration, for example, for oral or intravenous administration. The preparation may be in unit dosage form, for example, as tablets or capsules, or in unit or multiple dose ampoules or vials. From 0.1 to 10 mg of the active substance may be administered per kg body weight.

The following Examples illustrate the invention.

Example A

(1) 3-N-[2-[(2-Guanidino-4-thiazolyl)methylthio]-ethyl]-amino-4-methyl-5-amino-1,2,4,6-thiatriazine-1,1-dioxide

To a solution of 4-methyl-3-(4-methylphenoxy)-5-amino-1,2,4,6-thiatriazine-1,1-dioxide (100 mg) in 5 ml of methanol was added a solution of 2-guanidino-4-[(2-aminoethyl)thiomethyl]thiazole (90 mg) in 10 ml of ethanol. The resulting solution was kept at room temperature for 16 hours then refluxed for 4 hours. The solvent was evaporated under vacuum and the residual solid chromatographed on silica gel, eluting with a chloroform/methanol/30 % aqueous ammonia mixture to give the title compound as a colourless solid (45 mg).

'H n.m.r. (60 MHz) DMSO $\delta$ 2.62 (2 H,t), 3.20 (3 H,S), 3.34 (2H,t), 3.61 (2 H,S), 6.54 (1 H,S), 6.81 (4 H,br), 7.44 (1 H,br), 7.60 (1 H,br).

Using analogous reaction conditions the following compounds were prepared:

(2) 3-N-[2-[(2-Guanidino-4-thiazolyl)methylthio]-ethyl]-amino-4-ethyl-5-amino-1,2,4,6-thiatriazine-1,1-dioxide m.p. 134 - 6°C.

'H n.m.r. (250 MHz) DMSO $\delta$ 1.13 (3 H,t), 2.67 (2 H,t), 3.39 (2 H,M), 3.61 (2 H,S), 3.77 (2 H,q), 6.57 (1 H,S), 6.87 (5 H,brS,ex) 7.40 (2 H,brS,ex), 7.64 (1 H,t,ex).

(3) 3-N-[2-[(2-Guanidino-4-thiazolyl)methylthio]-ethyl]amino-4-(4-nitrobenzyl)-5-amino-1,2,4,6-thiatriazine-1,1-dioxide m.p. 160 - 2°C.

'H n.m.r. (250 MHz) DMSO δ 2.50 (2 H,t), 3.37 (2 H,t), 3.54 (2 H,S), 5.21 (2 H,S), 6.50 (1 H,S), 6.84 (6 H,brM), 7.45 and 8.24 (4 H,ABq) 7.46 (1 H,t,ex), 7.80 (1 H,t,ex).

The thiatriazines required as starting materials in example A are prepared by the following method:

Example B

(4) 3-(4-methylphenoxy)-4-methyl-5-amino-1,2,4,6-thiatriazine-1,1-dioxide

A suspension of 5-amino-3-(4-methylphenoxy) 4 H-1,2,4,6-thiatriazine-1,1,-dioxide (508 mg) in 10 ml of methanol was added to a stirred solution of sodium (46 mg) in 20 ml of methanol and the mixture stirred for 15 minutes. The resulting solution was evaporated to dryness and the residual salt suspended in acetonitrile (25 ml). Methyliodide was then added to the stirred suspension and the mixture refluxed for 3 hours, before evaporating to dryness under vacuum. The solid residue was taken up in water and extracted once with ethyl acetate. The ethyl acetate layer was dried and evaporated to a foam which was further purified by chromatography on silica gel, eluting with chloroform/methanol, to give the title compound (120 mg). m.p. 278 - 280°C.

'H n.m.r. (60 MHz) DMSO δ 2.18 (3 H,S), 3.27 (3 H,S), 7.05 (4 H,ABq), 7-60 (2 H,br).

Using analogous reaction conditions the following compounds were prepared:

(5) 3-(4-methylphenoxy)-4-ethyl-5-amino-1,2,4,6-thiatriazine-1,1-dioxide

m.p. 292 - 5°C.

'H n.m.r. (250 MHz) DMSO δ 1.29 (t,3 H), 2.33 (S,3 H), 4.01 (q, 2 H), 7.15 and 7.27 (ABq, 4 H), 7.88 (br.S,ex, 2 H).

(6) 3-(4-methylphenoxy)-4-(4-nitrobenzyl)-5-amino-1,2,4,6-thiatriazine-1,1-dioxide

m.p. 303 - 7°C.

$^1$H n.m.r. (250 MHz) DMSO $\delta$ 2.30 (3 H,S), 5.42 (2 H,S), 6.98 and 7.23 (4 H,ABq), 7.57 and 8.31 (4 H, ABq), 8.21 (2H, br.S,ex).

Example C

(7) 3,5-Bis-(4-methylphenoxy)-4-methyl-1,2,4,6-thiatriazine-1-oxide

To freshly prepared 4-methylphenylcyanate (13.3 g) was added bis (trimethylsilyl) methylamine (8.7 g) at room temperature in an inert atmosphere. The resulting mixture was kept at this temperature for 70 hours in a sealed flask. The yellow oil thus obtained was decanted from a small amount of white solid and dissolved in dichloromethane (25 ml), cooles to -5°C and stirred whilst thionyl chloride (2.0 ml) was added dropwise over 20 mins, not allowing the temperature to exceed 10°C. The solution was then stirred for 1 hr. at 10°C. Iced water (10 ml) was cautiously added and the layers separated, the organic phase washed with water (10 ml), dried ($MgSO_4$) and evaporated to give an oil. Trituration with ether affords 4-methyl-3,5-di-4'-methylphenoxy-1,2,4,6-thiatriazine-1-oxide as a white solid 3.82 g. Crystallisation from ethanol gave an analytically pure sample as needles m.p. 231-3°C.

$\gamma_{max}$($CHCl_3$) 1670(s), 1396(s), 1368(s), 1172(m), 1105(m) $cm^{-1}$

$^1$H n.m.r. (60 MHz) $CHCl_3$, $\delta$ 2.31 (6 H,s), 3.62 (3 H,s), 7.00 (8 H,ABq)

Example D

(8) 3,5-Bis-(4-methylphenoxy)-4-methyl-1,2,4,6-thiatriazine-1,1-dioxide

To a stirred solution of 4-methyl,3,5-di-4'-methylphenoxy-1,2,4,6-thiatriazine-1-oxide (5.0 g) in chloroform (20 ml) at room temperature a solution of m-chloroperbenzoic acid (3.5 g) in chloroform (10 ml) was added over 1 - 2 minutes. The exothermic reaction heats the mixture to reflux after a few minutes (cooling may be required) and a white solid precipitates. After heating at reflux for an additional 5 minutes the solid was filtered off and washed with a little cold chloroform to afford the title compound 4.4 g, m.p. 288 - 289°C.

$\gamma_{max}$ 1690(s), 1673(s), 1380(m), 1160(m), 831(m), 823(m) $cm^{-1}$

$^1H$ n.m.r. (60 MHz) DMSO $\delta$ 2.25 (6 H,S), 3.54 (3 H,S), 7.08 (8 H,ABq)

Example E

(9) 3-N-/2-/(2-Guanidino-4-thiazolyl)methylthio/ethyl/amino-4-methyl-5-(4-methylphenoxy)-1,2,4,6-thiatriazine-1,1-dioxide

To a stirred solution of 4-methyl,3,5-di-4'-methylphenoxy-1,2,4,6-thiatriazine-1,1-dioxide (4.4 g) in acetonitrile (25 ml) was added 2-guanidino-4-/(2-aminoethyl)thiomethyl/-thiazole (2.8 g) as a solution in ethanol (10 ml). The resulting mixture was stirred at room temperature for 6 hours, evaporated to dryness and chromatographed on silica gel. Elution with a chloroform/methanol/30 % aqueous ammonia mixture gave the title compound as a solid (4.4 g) m.p. 194 - 6°C.

$^1H$ n.m.r. (250 MHz) DMSO δ 2.33 (3 H,S), 2.67 (2 H,t), 3.43 (5 H,m), 3.67 (2 H,S), 6.71 (1 H,S), 7.15 - 7.29 (8 H, m inc 4 H,ex), 8.18 (1 H,t,ex).

Using analogous reaction conditions the following compounds were prepared:

(10) <u>3-N-[2-[(5-Methyl-4-imidazolyl)methylthio]ethyl]amino-4-methyl-5-(4-methylphenoxy)-1,2,4,6-thiatriazine-1,1-dioxide</u>

$^1H$ n.m.r. (60 MHz) DMSO δ 2.20 (3 H,S), 2.32 (3 H,S), 2.70 (2 H,t), 3.35 (2 H,t), 3.50 (3 H,S), 3,78 (2 H,S) 7.25 (4 H,ABq), 8.01 (1 H,S).

(11) <u>3-N-[2-[(5-Dimethylaminomethyl-2-furanyl)methylthio]-ethyl]amino-4-methyl-5-(4-methylphenoxy)-1,2,4,6-thiatriazine-1,1-dioxide</u>

$^1H$ n.m.r. (60 MHz) DMSO δ 2.20 (6 H,S), 2.30 (3 H,S), 2.70 (2 H,t), 3.35 (2 H,t), 3.48 (3 H,S), 3.50 (2 H,S), 3.80 (2 H,S), 6.20 (2 H,m), 7.25 (4 H,ABq)

(12) <u>3-N-[3-[3-(1-Piperidinylmethyl)phenoxy]propyl]amino-4-methyl-5-(4-methylphenoxy)-1,2,4,6-thiatriazine-1,1-dioxide</u>

$^1H$ n.m.r. (60 MHz) DMSO δ 1.55 (broad, 6 H), 2.00 (m, 2 H), 2.30 (m, 6 H), 2.32 (S, 3 H), 3.20 (S, 3 H), 3.50 (S, 2 H), 4.00 (t, 2 H), 7.10 (m, 8 H)

(13) <u>3-N-[2-[(2-Guanidino-4-thiazolyl)methylthio]ethyl]amino-4-methyl-5-(4-methylphenoxy)-1,2,4,6-thiatriazine-1-oxide</u>
m.p. 96 - 8°C

$^1H$ n.m.r. (250 MHz) DMSO δ 2.32 (3 H,S), 2.69 (2 H,m), 3.44 (3 H,S), 3.46 (2 H,m), 3.64 (2 H,S), 6.57 (1 H,S), 6.93 (4 H,brS, ex), 7.10 and 7.24 (4 H,ABq), 7.78 (1 H,t,ex)

Example F

(14) 3-N-/2-/(2-Guanidino-4-thiazolyl)methylthio/ethyl/amino-4-methyl-5-amino-1,2,4,6-thiatriazine-1,1-dioxide

Ammonia gas (anhydrous) was bubbled into a solution of E (9) (4.2 g) in ethanol/acetonitrile (1 : 1, 50 ml) for a period of 10 minutes at room temperature. The resulting solution was kept at this temperature for 24 hours then evaporated to dryness.

Chromatography of the crude product on silica gel eluting with a chloroform/methanol/30 % aqueous ammonia mixture gave 2.9 g of the title compound.

This material was dissolved in methanol with warming, and anhydrous hydrogen bromide gas was bubbled through the ice-cooled solution for 5 minutes. Nitrogen was then blown through the ice-cooled solution and the hydrobromide salt crystallised out. 2.27 g m.p. 248 - 250°C.

$\lambda_{max}$ 272 nm. E 10,000

$\nu_{max}$ (nujol) 3450 - 2500(B), 1655(m), 1605(m), 1375(m), 1135(m) $cm^{1}$

'H n.m.r. (60 MHz) $D_2O$ $\delta$ 2.70 (2 H,t), 3.27 (3 H,S), 3.37 (2 H,t), 3.73 (2 H,S), 6.98 (1 H,S)

Using analogous reaction conditions the following compounds were prepared:

(15) 3-N-/2-/(5-Methyl-4-imidazolyl)methylthio/ethyl/amino-4-methyl-5-amino-1,2,4,6-thiatriazine-1,1-dioxide

'H n.m.r. (60 MHz) DMSO $\delta$ 2.14 (3 H,S), 2.60 (2 H,t), 3.18 (3 H,S), 3.33 (2 H,t), 3.64 (2 H,S) 6.08 (1 H,br), 7.18 (1H,br), 7.54 (1 H,S)

(16) 3-N-/3-/3-(Dimethylaminomethyl)phenoxy7/propyl7/amino-4-methyl-5-amino-1,2,4,6-thiatriazine-1,1-dioxide

$^{1}$H n.m.r. (60 MHz) DMSO 1.98 (2 H,m), 2.13 (6 H,S), 3.30 (2 H,m), 3.33 (3 H,S), 4.04 (2 H,t), 6.9 (4 H,m)

(17) 3-N-/2-/(5-Dimethylaminomethyl-2-furanyl)methylthio7-ethyl7/amino-4-methyl-5-amino-1,2,4,6-thiatriazine-1,1-dioxide

$^{1}$H n.m.r. (60 MHz) DMSO 2.18 (6 H,S), 2.68 (2 H,t), 3.25 (3 H,S), 3.35 (2 H,t), 3.52 (2 H,S), 3.78 (2 H,S), 6.10 (2 H,m)

(18) 3-N-/3-/3-(1-Piperidinylmethyl)phenoxy7/propyl7/amino-4-methyl-5-amino-1,2,4,6-thiatriazine-1,1-dioxide

$^{1}$H n.m.r. (60 MHz) DMSO δ 1.43 (broad, 6 H), 1.95 (m, 2 H), 2.30 (m, 6 H), 3.20 (S, 3 H), 3.40 (S, 2 H), 3.95 (t, 2 H), 6.85 (m, 4 H)

Example G

Using the appropriate primary or secondary amine in place of the ammonia used in Example F, the compounds listed subsequently were prepared.

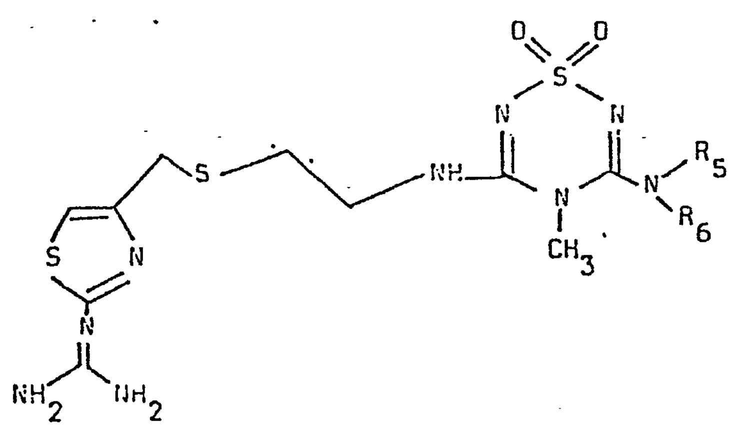

(19) $R_5$= H, $R_6$= $CH_3$, m.p. 135 - 7°C.

$^1$H n.m.r. (250 MHz) DMSO δ 2.62 (2 H,t), 2.70 (3 H,S), 3.19 (3 H,S), 3.38 (2 H,t), 3.61 (2 H,S), 6.56 (1 H,S), 6.83 (4 H,br), 7.49 (1 H,br), 7.61 (1 H,br).

(20) $R_5$= H, $R_6$= $CH_2CH_3$, m.p. 127 - 9°C.

$^1$H n.m.r. (250 MHz) DMSO δ 1.12 (3 H,t), 2.62 (2 H,t), 3.19 (2 H,q), 3.20 (3 H,S), 3.34 (2 H,t), 3.61 (2 H,S), 6.54 (1 H,S), 6.81 (4 H,br), 7.44 (1 H,br), 7.60 (1 H,br)

(21) $R_5$= H, $R_6$= $(CH_2)_2CH_3$, m.p. 202 - 3°C.

'H n.m.r. (δ rms= 0) (250 MHz) 0.87 (triplet, 3 H), 1.51 (sextet, 2 H), 2.61 (triplet, 2 H), 3.10 (quartet, 2 H), 3.20 (singlet, 3 H), 3.40 (triplet, 2 H), 3.60 (singlet, 2 H), 5.55 (singlet, 1 H), 5.83 (braod singlet, 4 H,ex), 7.46 (triplet, 1 H,ex) 7.62 (triplet, 1 H,ex)

(22) $R_5$= H, $R_6$= $(CH_2)_3CH_3$, m.p. 96 - 98°C.

'H n.m.r. (  rms= 0) (250 MHz) 0.85 (multiplet, 3 H), 1.28 (multiplet, 2 H), 1.46 (mulitplet, 2 H), 2.60 (triplet, 2 H), 3.14 (multiplet, 2 H), 3.19 (singlet, 3 H), 3.34 (triplet, 2 H), 3.60 (singlet, 2 H), 6.54 (singlet, 1 H), 6.83 (broad singlet, 4 H,ex) 7.46 (very broad singlet, 2 H,ex)

(23) $R_5$= H, $R_6$= $(CH_2)_4CH_3$, m.p. 124 - 5°C.

'H n.m.r. (  rms= 0) (250 MHz) 0.87 (triplet, 3 H), 1.28 (mulitplet, 4 H), 1.51 (pentet, 2 H), 2.61 (triplet, 2 H), 3.13 (multiplet, 2 H),

3.21 (singlet, 3 H), 3.36 (triplet, 2 H), 3.64 (singlet, 2 H), 6.69 (singlet, 1 H), 7. 13 broad singlet, 4 H,ex), 7.48 (triplet, 1 H, ex), 7.67 (triplet, 1 H,ex)

(24) $R_5$= $CH_3$, $R_6$= $CH_3$, m.p. 120 - 2°C.

'H n.m.r. (  rms= 0) (250 MHz) 2.66 (triplet, 2 H), 2.88 (singlet, 6 H), 3.33 (singlet, 3 H), 3.38 (triplet, 2 H), 3.66 (singlet, 2 H), 6.62 (singlet, 1 H), 6.94 (broad singlet, 4 H,ex), 7.84 (triplet, 1 H,ex)

(25) $R_5$= H, $R_6$= $CH(CH_3)_2$, m.p. 139 - 141°C.

'H n.m.r. (  rms= 0) (250 MHz) 1.16 (doublet, 6 H), 2.62 (triplet, 2 H), 3.21 (singlet, 3 H), 3.36 (triplet, 2 H), 3.61 (singlet, 2 H), 3.90 (multiplet, 1 H), 6.56 (singlet, 1 H), 6.83 (broad singlet, 4 H,ex), 7.14 (singlet, 1 H,ex), 7.60 (broad singlet, 1 H,ex)

(26) $R_5$= H, $R_6$= $CH_2CH{=}CH_2$, m.p. 204 - 205°C.

'H n.m.r. (  rms= 0) (250 MHz) 2.67 (triplet, 2 H), 3.26 (singlet, 3 H), 3.38 (triplet, 2 H), 3.65 (singlet, 2 H), 3.82 (doublet of triplets, 2 H), 5.19 (quartet of quartets, 2 H), 5.90 (multiplet, 1 H), 6.60 (singlet, 1 H), 6.86 (broad singlet, 4 H,ex), 7.70 (singlet, 1 H,ex)

(27) $R_5$= H, $R_6$= $CH_2C{\equiv}CH$, m.p. 210 - 2°C.

'H n.m.r. ( rms= 0) (250 MHz) 2.63 (triplet, 2 H), 3.21 (singlet, 3 H), 3.23 (triplet, 1 H), 3.36 (triplet, 2 H), 3.62 (singlet, 2 H), 3.97 (doublet, 2 H), 6.58 (singlet, 1 H), 6.87 (broad singlet, 4 H,ex), 7.72 (triplet, 1 H,ex), 7.78 (triplet, 1 H,ex)

(28) $R_5$= H, $R_6$= $CH_2CH_2OCH_3$, m.p. 113 - 5°C.

'H n.m.r. ( rms= 0) (250 MHz) 2.60 (triplet, 2 H), 3.18 (singlet, 3 H), 3.25 (singlet, 3 H), 3.36 (quartet, 4 H), 3.46 (triplet, 2 H), 3.60 (singlet, 2 H), 6.54 (singlet, 1 H), 6.82 (broad singlet, 4 H,ex), 7.55 (singlet, 1 H,ex), 7.65 (singlet, 1 H,ex)

(29) $R_5$= H, $R_6$= 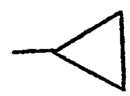 , m.p. 139 - 143°C.

'H n.m.r. ( rms= 0) (250 MHz) 0.55 (multiplet, 2 H), 0.71 (multiplet, 2 H), 2.62 (triplet, 3 H), 3.16 (singlet, 3 H), 3.34 (triplet, 2 H), 3.61 (singlet, 2 H), 6.56 (singlet, 1 H), 5.83 (broad singlet, 4 H,ex), 7.53 (singlet, 1 H,ex), 7.63 (triplet, 1 H,ex)

(30) $R_5$= H, $R_6$= 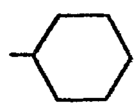 , m.p. 136 - 8°C.

'H n.m.r. ( rms= 0) (250 MHz) 1.28 (triplet, 4 H), 1.69 (multiplet, 6 H), 2.63 (triplet, 2 H), 3.21 (singlet, 3 H), 3.36 (triplet, 2 H), 3.60 (multiplet, 1 H), 3.62 (singlet, 2 H), 6.57 (singlet, 1 H), 6.85 (broad singlet, 4 H,ex), 7.14 (singlet, 1 H,ex), 7.66 8v. broad singlet, 1 H,ex)

(31) $R_5$, $R_6$= $-CH_2CH_2CH_2CH_2CH_2-$, m.p. 220 - 2°C.

'H n.m.r. ( rms= 0) (250 MHz) 1.58 (singlet, 6 H), 2.63 (triplet, 2 H), 3.20 (singlet, 4 H), 3.28 (singlet, 3 H), 3.37 (triplet, 2 H), 3.62 (singlet, 2 H), 6.55 (singlet, 1 H), 6.83 (broad singlet, 4 H,ex), 7.80 (broad singlet, 1 H,ex)

(32) $R_5$= H, $R_6$= $CH_2$ 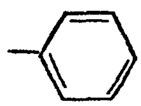 , m.p. 112 - 4°C.

'H n.m.r. ( rms= 0) (250 MHz) 2.62 (triplet, 2 H), 3.26 (singlet, 3 H), 3.34 (triplet, 2 H), 3.60 (singlet, 2 H), 4.40 (singlet, 2 H), 6.55 (singlet, 1 H), 6.84 (broad singlet, 4 H,ex), 7.31 (multiplet, 5 H), 7.70 (v. broad singlet, 1 H,ex), 8.06 (v. broad singlet, 1 H,ex)

(33) $R_5$ = H, $R_6$ = $CH_2CH_2OH$, mp. 209 - 211°C

2.64 (2H, t), 3.22 (3H, s), 3.28 (2H, t), 3.39 (2H, t), 3.56 (2H, t), 3.65 (2H, s), 4.82 (1H, br.s, ex), 6.60 (1H, s), 6.87 (4H, br.s, ex), 7.58 (2H, v.br.s, ex).

(34) $R_5$ = H, $R_6$ = $CH_2CH_2CH_2OH$, mp. 189 - 90°C

1.68 (2H, m), 2.62 (2H, t), 3.22 (3H, s), 3.28 (2H, t), 3.42 (2H, t), 3.46 (2H, t), 3.62 (2H, s), 4.54 (1H, br.s, ex), 6.57 (1H, s), 6.84 (4H, br.s, ex), 7.48 (2H, br, s, ex).

(35) $R_5$ = H, $R_6$ = $CH(CH_3)CH_2CH_3$, mp. 134-6°C

0.87 (3H, t), 1.13 (3H, d), 1.53 (2H, m), 2.64 (2H, t), 3.24 (3H, s), 3.40 (2H, t), 3.63 (2H, s), 3.74 (1H, m), 6.58 (1H, s), 6.85 (4H, br. s, ex), 7.11 (1H, d, ex), 7.65 (1H, br. s, ex).

(36) $R_5$ = H, $R_6$ = $CH_2$ 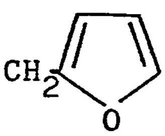 , mp. 109-111°C

2.61 (2H, t), 3.22 (3H, s), 3.37 (2H, t), 3.61 (2H, s), 4.38 (2H, d), 6.34 and 6.42 (2H, m), 6.57 (1H, s), 6.84 (4H, br. s, ex), 7.60 (1H, br, s), 7.69 (1H, t, ex), 8.01 (1H, t, ex).

(37) $R_5$ = H, $R_6$ = $CH_2$ 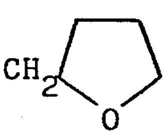 , mp. 100-2°C

1.42-2.00 (4H, m), 2.62 (2H, t), 3.20 (3H, s), 3.33 (2H, t), 3.61 (2H, s), 3.63 (2H, m), 3.75 (2H, m) 4.00 (1H, m), 6.57 (1H, s), 6.84 (4H, br, s, ex), 7.50 (2H, v. br. s., ex).

(38) $R_5$ = H, $R_6$ = $CH_2$ 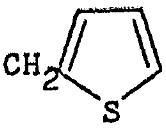 , mp. 116-8°C

2.63 (2H, t), 3.22 (3H, s), 3.40 (2H, t), 3.61 (2H, s), 4.54 (2H, d), 6.57 (1H, s), 6.86 (4H, br. s, ex), 6.98 (1H, dd), 7.06 (1H, d), 7.42 (1H, dd), 7.68 (1H, t, ex). 8.14 (1H, t, ex).

(39) $R_5$ = H, $R_6$ = $CH_2$ 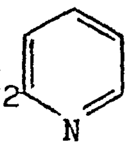 , mp. 111-2°C

2.62 (2H, t), 3.33 (3H, s), 3.42 (2H, t), 3.62 (2H, s), 4.48 (2H, br. s), 6.56 (1H, s), 6.84 (4H, br. s, ex), 7.28 (1H, m), 7.37 (1H, d), 7.75 (2H, m, (1H,ex)), 8.12 (1H, t, ex). 8.51 (1H, m).

(40) $R_5$ = H, $R_6$ = $CH_2$ 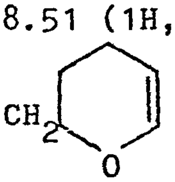 , mp. 120-2°C

1.52 (1H, m), 2.00 (3H, m), 2.63 (2H, t), 3.23 (3H, s), 3.40 (4H, m), 3.67 (2H, s), 3.99 (1H, q), 4.72 (1H, br.m), 6.40 (1H, d), 6.77 (1H, s), 7.26 (4H, br.s, ex), 7.73 (2H, q, ex).

(41) $R_5$ = H, $R_6$ = $CH_2$ 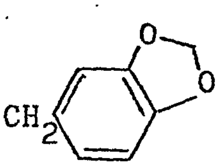 , mp. 105-6°C

2.63 (2H, t), 3.24 (3H, s), 3.43 (2H, t), 3.61 (2H, s), 4.29 (2H, s), 5.99 (2H, s), 6.57 (1H, s), 6.78-6.94 (7H,m, (4H, ex)), 7.75 (1H, br.s,ex), 8.00 (1H, br.s,ex).

(42) $R_5$ = H, $R_6$ = $CH_2$-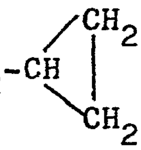 , mp. 118-20°C

0.23 (2H, m), 0.43 (2H, m), 1.05 (1H, m), 2.62 (2H, t), 3.02 (2H, d), 3.22 (3H, s), 3.36 (2H, t), 3.61 (2H, s), 6.57 (1H, s), 6.86 (4H, br.s, ex), 7.70 (2H, v.br.s,ex).

(43) $R_5$ = H, $R_6$ = $CH_2CH_2CH_2N(CH_3)_2$, mp. 199-200°C

1.66 (2H, m), 2.16 (6H, s), 2.28 (2H, t), 2.64 (2H, t), 3.31 (3H, s), 3.21 (2H, m, superimposed), 3.40 (2H, t), 3.64 (2H, s), 3.57 (1H, s), 6.88 (4H, br.s, ex), 7.72 (2H, v.br.m, ex).

(44) $R_5$ = H $R_6$ = $CH_2CH_2N(CH_3)_2$, mp. 96-8°C

2.24 (6H, s), 2.50 (2H partly obscured by solvent), 2.62 (2H, t), 3.20 (3H, s) 3.32 (4H, m), 3.61 (2H, s), 6.57 (1H, s), 6.84 (4H, br.s, ex), 7.44 (1H, br.s, ex), 7.70 (1H, t, ex).

(45) $R_5$ = H, $R_6$ = $CH_2CH_2NHCH_3$, mp. 157-9°C

1.60 (1H, v.br.s, ex), 2.27 (3H, s), 2.61 (4H, m), 3.20 (3H, s), 3.27 (2H, t), 3.37 (2H, t), 3.61 (2H, s), 6.57 (1H,s), 6,85 (4H, br.s, ex), 7.06 (1H, v.br.s,ex), 7.64 (1H, br.s, ex).

(46) $R_5$ = H, $R_6$ = $CH_2CH_2NH_2$, mp. 138-40°C

spectrum run in $d_6$-DMSO + 5 % $D_2O$
2.58 (2H, t), 2.67 (2H, t), 3.18 (3H, s), 3.21 (2H, t), 3.34 (2H, t), 3.58 (2H, s), 6.57 (1H, s).

(47) $R_5$ = H, $R_6$ = $CH_2CH_2$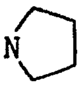 , mp. 218-224°C

1.91 (4H, br.s), 2.65 (2H, t), 3.11 (6H, m), 3.29 (3H, s), 3.44 (2H, m), 3.54 (2H, m), 3.63 (2H, s), 6.63 (1H, s), 6.93 (4H, br.s, ex), 7.85 (2H, t, ex).

(48) $R_5$ = H, $R_6$ = $CH_2CH_2$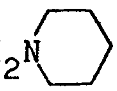 , mp. 198-200°C

1.45 (2H, br.s), 1.66 (4H, br.s), 2.62 (2H, t), 2.90 (6H, br.s), 3.23 (3H, s), 3.37 (2H, t), 3.45 (2H, m), 3,61 (2H, s), 6.59 (1H, s), 6.94 (4H, br.s, ex), 7.78 (1H, br.s, ex), 7.82 (1H, t, ex).

(49) $R_5$ = H, $R_6$ = $CH_2$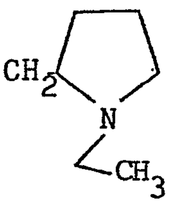 , mp. 108-10°C

1.02 (3H, t), 1.50-1.88 (4H, m), 2.14 (2H, m), 2.28 (2H, m), 2.62 (2H, t), 2.76 (1H, m), 3.00 (2H, m), 3.20 (3H, s), 3.36 (2H, m), 3.62 (2H, s), 6.58 (1H, s), 6.85 (4H, br.s, ex), 7.36 (1H, v.br.s, ex), 7.64 (1H, br.s, ex).

(50) $R_5$ = H, $R_6$ = $(CH_2)_5CH_3$ , mp. 89-91°C

'Hnmr (250MHz), DMSO $\delta$, 0.87 (3H, m), 1.27 (6H, m), 1.50 (2H, m), 2.62 (2H, t). 3.14 (2H, t), 3.20 (3H, s), 3.36 (2H, m), 3.61 (2H, s), 6.57 (1H, s), 6.86 (6H, broad s, ex).

(51) $R_5$ = H, $R_6$ = 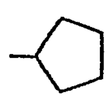 , mp. 79-81°C

'Hnmr (250 MHz), DMSO $\delta$, 1.51 (4H, m), 1.67 (2H, m), 1.88 (2H, m), 2.63 (2H, t), 3.21 (3H, s), 3.32 (2H, m), 3.61 (2H, s), 3.98 (1H, m), 6.57 (1H, s), 6.86 (4H, broad s, ex), 7.19 (1H, m ex), 7.65 (1H, broad s, ex).

(52) $R_5$ = H, $R_6$ = 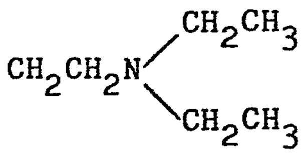 , mp. 110-111°C

'Hnmr (250 MHz), DMSO , 0.96 (6H, t), 2.48 (4H, q), 2.52 (2H, t), 2.62 (2H, t), 3.19 (5H, m), 3.35 (2H, m), 3.61 (2H, s), 6.57 (1H, s), 6.85 (4H, broad s, ex), 7.39 (1H, broad s, ex), 7.63 (1H, broad s, ex).

(53) $R_5$ = H, $R_6$ = $CH_2\overset{\overset{O}{\|}}{C}-OCH_2CH_3$ , mp. foam

'Hnmr (250 MHz), DMSO δ, 1.21 (3H, t), 2.64 (2H, t), 3.27 (3H, s), 3.39 (2H, m), 3.66 (2H, s), 3.92 (2H, d), 4.13 (2H, quartet), 6.74 (1H, s), 7.21 (4H, broad s, ex), 7.82 (1H, t, ex), 8.08 (1H, t, ex).

(54) $R_5$ = H, $R_6$ = 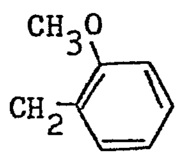 , mp. 148-150°C

'Hnmr (250 MHz), DMSO δ 2.64 (2H, t), 3.29 (3H, s), 3.37 (2H, m), 3.61 2H, s), 3.82 (3H, s), 4.36 (2H, s), 6.57 (1H, s), 6.76-7.31 (8H, m inc. 4H, ex), 7.71 (1H, broad s, ex), 7.87 (1H, broad s, ex).

(55) $R_5$ = H, $R_6$ = 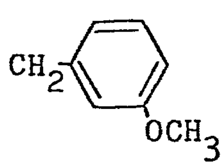 , mp. 114-115°C

'Hnmr (250 MHz), DMSO δ, 2.63 (2H, t), 3.27 (3H, s), 3.40 (2H, m), 3.61 (2H, s), 3.74 (3H, s), 4.37 (2H, s), 6.57 (1H, s), 6.81-7.28 (8H, m inc. 4H ex), 7.68 (1H, broad s, ex), 8.02 (1H, broad s, ex).

(56) $R_5$ = H, $R_6$ = 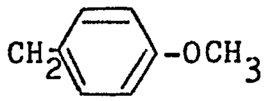 , mp. 118-120°C

'Hnmr (250 MHz), DMSO δ, 2.63 (2H, t), 3.25 (3H, s), 3.37 (2H, m), 3.61 (2H, s), 3.73 (3H, s), 4.32 (2H, s), 6.57 (1H, s), 6.89 (2H, broad s, ex), 6.90 and 7.27 (4H, ABq), 7.7 (2H, broad s, ex), 8.01 (2H, broad s ex).

(57) $R_5$ = H, $R_6$ = 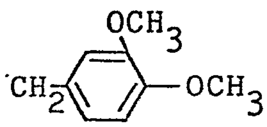 , mp. 159-160°C

'Hnmr (250 MHz), DMSO δ 2.63 (2H, t), 3.25 (3H, s), 3.37 (2H m), 3.61 (2H, s), 3.74 (3H, s), 3.76 (3H, s), 4.31 (2H, s), 6.58 (1H, s), 6.83 - 7.02 (7H, m inc. 4H ex), 7.73 (1H, broad s, ex), 8.01 (1H, broad s, ex).

(58) $R_5$ = H, $R_6$ = 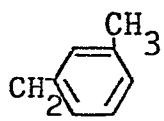 , mp. 129-130°C

'Hnmr (250 MHz), DMSO δ, 2.30 (3H, s), 2.64 (2H, t), 3.27 (3H, s), 3.40 (2H, m), 3.62 (2H, s), 4.37 (2H, s), 6.57 (1H, s), 6.84 (4H, broad s ex), 7.06-7.27 (4H, m), 7.71 (1H, broad s, ex), 8.01 (1H, broad s, ex).

(59) $R_5$ = H, $R_6$ = 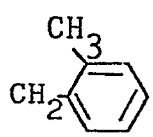 , mp. 140-141°C

'Hnmr (250 MHz), DMSO δ, 2.30 (3H, s), 2.64 (2H, t), 3.28 (3H, s), 3.39 (2H, m), 3.62 (2H, s), 4.36 (2H, s), 6.57 (1H, s), 6.86 (4H, broad s, ex), 7.18 (4H, m), 7.69 (1H, broad s, ex), 7.91 (1H, broad s, ex).

(60) $R_5$ = H, $R_6$ = 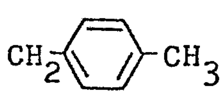 , mp. 129-130°C

'Hnmr (250 MHz), DMSO δ, 2.28 (3H, s), 2.64 (2H, t), 3.26 (3H, s), 3.38 (2H, m), 3.62 (2H, s), 4.35 (2H, s), 6.56 (1H, s), 6.83 (4H, broad s, ex), 7.08-7,24 (4H, ABq), 7.80 (2H, broad s, ex).

(61) $R_5$ = H, $R_6$ = $CH_2$- 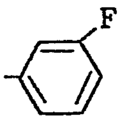 ,

'Hnmr (250 MHz), DMSO δ, 2.64 (2H, t), 3.28 (3H, s), 3.40 (2H, m), 3.62 (2H, s), 4.42 (2H, s), 6.57 (1H, s), 6.84 (4H, broad s, ex), 7.05-7.43 (4H, m), 7.69 (1H, broad s, ex), 8.07 (1H, broad s, ex).

(62) $R_5$ = H, $R_6$ = $CH_2$ 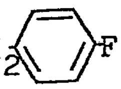 , mp. 127-128°C

'Hnmr (250 MHz), DMSO δ, 2.63 (2H, t), 3.26 (3H, s), 3.39 (2H, m), 3.61 (2H, s), 4.38 (2H, s), 6.57 (1H, s), 6.86 (4H, broad s, ex) 7.13-7.42 (4H, m), 7.72 (1H, broad s, ex), 8.08 (1H, broad s, ex).

(63) $R_5$ = H, $R_6$ = $CH_2$ 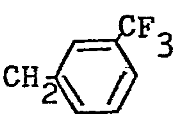 , mp.

'Hnmr (250 MHz), DMSO δ, 2.64 (2H, t), 3.28 (3H, s), 3.40 (2H, m), 3.62 (2H, s), 4.49 (2H, d), 6.57 (1H, s), 6.83 (4H, broad s, ex) 7.58-7.71 (5H, m inc. 1H ex), 8.11 (1H, t, ex).

(64) $R_5$ = H, $R_6$ = $CH_2$ 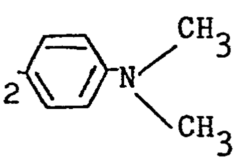 , mp. 228-230°C.

'Hnmr (250 MHz), DMSO δ, 2.63 (2H, t), 2.86 (6H, s), 3.23 (3H, s), 3.40 (2H, m), 3.61 (2H, s), 4.26 (2H, s), 6.57 (1H, s), 6.69 and 7.17 (4H, ABq), 6.84 (4H broad s, ex), 7.66 (1H, broad s, ex), 7.90 (1H, broad s,ex).

(65) $R_5$ = H, $R_6$ = $(CH_2)_2$- 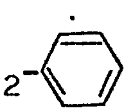 , mp. 214-215°C

'Hnmr, (250 MHz), DMSO, δ, 2.63 (2H, t), 2.85 (2H, t), 3.16 (3H, s), 3.39 (4H, m), 3,62 (2H, s), 6.58 (1H, s), 6.86 (4H, broad s, ex), 7.27 (5H, m), 7.63 (2H, broad s, ex).

(65a) $R_5$ = H, $R_6$ = C≡N ,

'Hnmr (250 MHz), DMSO $\delta$, 2.64 (2H, t), 3.24 (3H, s), 3.34 (2H, m), 3.62 (2H, s), 6.56 (1H, s), 6.57 (1H, broad s, ex), 6.82 (4H, broad s, ex), 7.94 (1H, broad s, ex).

(65b) $R_5$ = H, $R_6$ = $CH_2CH_2$ 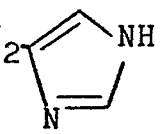 mp. 216-18°C

$^1$H n.m.r. (250 MHz) $D_2O$, $\delta$ 2.72 (2H, t), 2.95 (2H, t), 3.20 (3H, s), 3.42 (2H, t), 3.60 (2H, t), 3.72 (2H, s), 6.84 (1H, s), 7.11 (1H, s), 8.16 (1H, s).

(66) 3N-[3-[3-(1-Piperidinylmethyl)phenoxy] propyl] amino-4-methyl-5-(2-dimethylamino ethyl)amino-1,2,4,6-thiatriazine-1,1-dioxide.

$^1$ H n.m.r. (250 MHz) DMSO, $\delta$, 1.45 (m, 6H), 2.07 (m, 2H), 2.14 (2, 6H), 2.34 (s, 4H), 2.40 (t, 2H), 3.24 (s, 3H), 3.28 (m, 4H), 3.40 (s, 2H), 4.02 (t, 2H), 6.85 (m, 4H), 7.21 (t, 1H).

<u>Example H</u>

(67) <u>3-N[2-[(2-Guanidino-4-thiazolyl)methylthio] ethyl] amino-4-methyl-5-methylamino-1,2,4,6-thiatriazine-1-oxide</u>

This compound was prepared by procedures analogous to those given in examples C, E and F above with omission of the oxidation step given in example D.

m.p. 98-101°C.

'H n.m.r. (60MHz) DMSO δ2.55 (2H, t), 2.60 (3H, d), 3.12 (3H, s), 3.35 (2H, t), 3.56 (2H, s), 6.50 (1H, s).

Also prepared by this method was

(68) 3-N-[2-[(2-Guanidino-4-thiazolyl)methylthio]ethyl] amino-4-methyl-5-amino-1,2,4,6-thiatriazine-1-oxide.

m.p. 171-3°C.

'H n.m.r. (250 MHz) DMSO δ, 2.64 (2H, m), 3.19 (3H, s), 3.38 (2H, m), 3.62 (2H, s), 6.56 (1H, s), 6.89 (6H, br.s, ex), 7,22 (1H, t, ex).

Example I

(69) 3-N-[2-((2-Guanidino-4-thiazolyl) methylthio)ethyl) amino-4H-5-amino-1,2,4,6-thiatriazine-1,1-dioxide

To a solution of 3-N[2-((2-Guanidino-4-thiazolyl)methylthio)ethyl)amino-4-(4-nitrobenzyl)-5-amino-1,2,4,6-thiatriazine-1,1-dioxide (200 mg) in ethyl acetate (30 ml) aqueous sodium carbonate solution (24 mg in water, 10 ml) and 10 % Palladium on charcoal (200 mg) were added. This mixture was hydrogenated at 50 p.s.i. for 3hrs on a Parr hydrogenator. The reaction mixture was filtered through "Hyflow" (filter aid) and the layers separated and the aqueous layer freeze dried to give the title compound (114 mg) as the sodium salt.

'H n.m.r. (250 MHz) $D_2O$ δ, 2.72 (2H, t), 3.41 (2H, t), 3.76 (2H, s), 6.76 (1H, s).

$^{13}C$ n.m.r. (250 MHz), $D_2O$/DMSO, δ 32.03, 32.40, 40.68, 108.60, 149.24, 158.50, 161.20, 163.63, 175.66.

0104611

Example J

(70) 3-N-[2-[(2-Guanidino-4-thiazolyl) methylthio] ethyl] amino-4-phenyl-5-methyl-1,2,4,6-thiatriazine-1,1-dioxide

To a stirred solution of 53 mg of 2-Guanidino-4-[(2-aminoethyl)thiomethyl]thiazole in 5 ml of ethanol was added dropwise in 5 ml of dry acetonitrile 51 mg of 3-chloro-4-phenyl-5-methyl-1,2,4,6 thiatriazine-1,1-dioxide. The reaction was stirred for 30 minutes at room temperature and then the solvent evaporated in vacuo. The product was purified by column chromatography on silica gel, eluting with chloroform/methanol 9:1, to yield 76 mg of the title compound as an amorphous solid.

'H n.m.r. (250 MHz) DMSO $\delta$, 1.80 (S, 3H), 2.53 (t, 2H), 3.28 (m, 2H), 3.60 (s, 2H), 6.70 (s, 1H), 7.52-7.63 (m, 5H).

Using analogous reaction conditions the following compounds were prepared:

(71) 3-N-[2-[(2-Guanidino-4-thiazolyl)methylthio]ethyl] amino-4-methyl-5-phenyl-1,2,4,6-thiatriazine-1,1-dioxide

m.p. 238-40°C (decomp.)

'H n.m.r. (250 MHz) DMSO $\delta$, 2.70 (t, 2H), 3.20 (s, 3H), 3.46 (m, 2H), 3.67 (s, 2H), 6.65 (s, 1H), 7.5-7.67 (m, 5H).

(72) 3-N-[2-[(2-Guanidino-4-thiazolyl)methylthio]ethyl]amino-4-methyl-5-methyl-1,2,4,6-thiatriazine-1,1-dioxide

'H n.m.r. (250 MHz) DMSO $\delta$, 2.33 (3H, s), 2.63 (2H t), 3.29 (3H, s), 3.39 (2H, m), 3,78 (2H, s), 7.19 (1H, s), 7.99 (1H, t, ex), 8.21 (4H, br. s. ex).

The chlorothiatriazines required as starting materials in example I are prepared by the following methods ( Example K ):

Example K

(73) 3-Chloro-4-phenyl-5-methyl-1,2,4,6-thiatriazine-1,1-dioxide

54 mg of 3-methylthio-4-phenyl-5-methyl-1,2,4,6-thiatriazine-1,1-dioxide was suspended in 5 ml of dry ethylacetate with stirring and chlorine gas bubbled slowly trough until the solid had dissolved. Some cooling was required to maintain the reaction at room temperature. Excess chlorine was removed by concentration of the ethyl acetate solution in vacuo and the product was crystallized by addition of hexane. The crystalline solid was collected and recrystallized from ethyl acetate/hexane as white needles, yielding 32 mg of the title compound, m.p. 198-200°C.

'H n.m.r. (250 MHz) $CDCl_3$, $\delta$, 2.07 (s, 3H) 7.36-7.62 (m, 5H).

Mass Spec. 259 (M+2), 257 (m/e), 216 ($M-CH_3CN$), 117 ($M-C_6H_5NCN$)

0104611

Also prepared using this procedure:

(74) 3-Chloro-4-methyl-5-methyl-1,2,4,6-thiatriazine-1,1-dioxide

'H n.m.r. (250 MHz) DMSO $\delta$, 2.40 (3H, s), 3.28 (3H, s).

Example L

(75) 3-Chloro-4-methyl-5-phenyl-1,2,4,6-thiatriazine-1,1-dioxide

100 mg of 3-methylthio-4-methyl-5-phenyl-1,2,4,6-thiatriazine-1,1-dioxide was suspended in a solution of 5 mg anhydrous zinc chloride in 10 ml ethyl acetate. Chlorine gas was bubbled slowly through until a clear solution had formed, while maintaining the reaction at ambient temperature with some cooling. Excess chlorine and some of the solvent was removed under vacuum, and the product was crystallized by addition of hexane, yielding 74 mg of the title compound as white needles.

'H n.m.r. (60 MHz), $CDCl_3$/acetone-$d_6$, 3.63 (s, 3H), 7.60 (broad s, 5H).

Example M

(76) 3-Methylthio-4-phenyl-5-methyl-1,2,4,6-thiatriazine-1,1-dioxide

2.8 g of 1-phenyl-2-methyl-3-(N'-acetyl)sulphamoyl-isothiourea was dissolved in 50 ml of diglyme and refluxed for 5 hours under a nitrogen atmosphere. The solution was filtered through cotton wool, and the solvent evaporated in vacuo. The residual solid was crystallized from ethanol/hexane. yielding 370 mg of the title compound, m.p. 295-99°C (decomp.)

'H n.m.r. (250 MHz) $CDCl_3$, $\delta$, 1.90 (s, 3H), 2.30 (s, 3H), 7.58-7.71 (m, 5H).

Using this procedure the following were prepared:

(77) 3-Methylthio-4-methyl-5-phenyl-1,2,4,6-thiatriazine-1,1-dioxide

m.p. 188-90°C

'H n.m.r. (250 MHz) $CDCl_3$, δ, 2.58 (s, 3H), 3.41 (s, 3H), 7.55 (m, 5H).

(78) 3-Methylthio-4-methyl-5-methyl-1,2,4,6-thiatriazine-1,1-dioxide

m.p. 233-235°C

'H n.m.r. (250 MHz) DMSO, δ, 2.38 (3H, s), 2.46 (3H, s), 3.45 (3H, s).

Example N

(79) 1-Phenyl-2-methyl-3-(N'-acetyl)sulphamoyl isothiourea

2.23 g of 1-acetyl-1-phenyl-2-methylisothiourea (See: H. Wheeler, Am. Chem. J., 27, 270 (1902) was dissolved in 50 ml of dry acetonitrile and stirred at room temperature. 1.25 g of triethylamine in 10 ml of dry acetonitrile and 1.47 g of aminosulphonyl chloride in 10 ml of dry acetonitrile were added dropwise simultaneously. The reaction was left to stir for 30 minutes, then evaporated to dryness in vacuo. The residue was partitioned between dichloromethane and water, and the aqueous layer extracted once again with dichloromethane. The combined organic layers were washed once with water, dried over $MgSO_4$ and evaporated, to give the title compound as a white crystalline solid: 2.8 g.

m.p. 144-148°C.

'H n.m.r. (250 MHz) $CDCl_3$, δ, 2.18 (s, 3H), 2.36 (s. 3H), 7.32-7.45 (m, 5H).

Using this procedure the following were obtained:

(80) 1,2-Dimethyl-3-(N'-benzoyl)sulphamoyl isothiourea

'H n.m.r. (60 MHz) $CDCl_3$, $\delta$, 2.37 (s, 3H), 3.10 (d, 3H), 7.3-8.1 (m, 5H).

The n.m.r. and thin layer chromatography of the crude product from this preparation indicated the presence of some cyclized thiatriazine. The crude material was converted cleanly to the thiatriazine by heating according to example M.

(81) 1,2-Dimethyl-3-(N'-acetyl)-sulfamoyl isothiourea

'H n.m.r. (60 MHz) $CDCl_3$, $\delta$, 2.14 (3H, s), 2.44 (3H, s), 3.06 (3H, d).

Example O

(82) 1-Benzoyl-1,2-dimethylisothiourea

2.32 g of N,S-dimethyl isothiourea hydriodide salt was stirred in suspension in 40 ml of tetrahydrofuran at -20°C. 2.1 g of triethylamine was added, followed by dropwise addition of 1.4 g of benzoyl chloride. The reaction was stirred for 30 minutes at -20°C and then allowed to warm to ambient. The solvent was evaporated in vacuo and the residue purified by column chromatography on silicagel, eluting with ethyl acetate-hexane to yield 1.1 g of the title compound as a gum.

'H n.m.r. (60 MHz) $CDCl_3$, $\delta$, 2.23 (s, 3H), 3.33 (s, 3H), 7.1-7.6(m, 5H)

The isomeric 1,2-dimethyl-3-benzoylisothiourea was obtained as a 1:1 mixture with the above by carrying out the above procedure at ambient temperature in dichlormethane as solvent:

'H n.m.r. (60 MHz) $CDCl_3$, $\delta$, 2.62 (s, 3H), 3.02 (d, 3H), 7.15-8.30 (m, 5H).

(83) 1-Acetyl-1,2-dimethylisothiourea

'H n.m.r. (60 MHz) DMSO, , 2.48 (3H, s), 2.66 (3H, s), 3.45 (3H, s).

Example P

(84) 3-N /2-((2-Guanidino-4-thiazolyl_7methylthio)ethyl) amino-4-methyl-5-ethoxy-1,2,4,6-thiatriazine-1,1-dioxide

To a solution of sodium (0,23 g) in ethanol (100 ml) 3 N/ 2-((2-guanidino-4-thiazolyl)methylthio)7ethyl)amino-4-methyl-5-(4-methylphenoxy)-1,2,4,6-thiatriazine-1,1-dioxide(5.00 g) was added and the mixture stirred and heated at reflux for 1 hour. The reaction mixture was cooled and evaporated to dryness and the residue chromatographed on silica gel. Elution with chloroform/ methanol mixtures gave the title compound (3.21 g) as white solid m.p. 188-190°C.

'H n.m.r. (60 MHz) DMSO, $\delta$, 1.23 (3H, q), 2.61 (2H, t), 3.22 (3H, s), 3.35 (3H, m), 3.60 (2H, s), 4.47 (2H, q), 6.50 (1H, s), 6.73 (3H, br.s, ex), 7.75 (1H, br. s, ex).

Claims

1. A compound of the general formula I

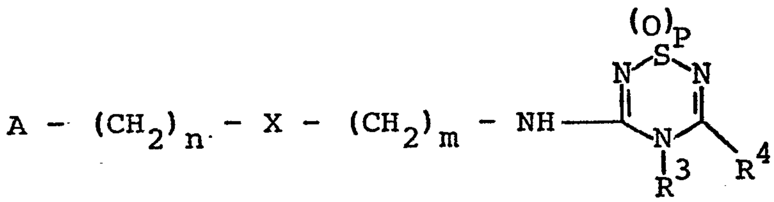 (I)

and its physiologically tolerable salts, in which formula

A represents a phenyl, imidazolyl, thiazolyl, furyl, thienyl, or pyridyl radical; which radical may contain one or two substituents, the first being selected from $(C_1-C_4)$ alkyl groups, and the second from $(C_1-C_4)$ alkyl, guanidino, and $-CH_2NR^1R^2$ groups, $R^1$ and $R^2$, which may be the same or different, each representing a hydrogen atom or a $(C_1-C_6)$alkyl group, or together with the nitrogen atom to which they are attached, may form a pyrrolidine, piperidine, morpholine, or N-methylpiperazine ring;

X represents -O-, -S-, or $-CH_2$;

n represents 0 or 1;

m represents 2 or 3;

p represents 1 or 2;

$R^3$ represents a hydrogen atom or a $(C_1-C_6)$alkyl, $(C_2-C_6)$alkenyl, $(C_3-C_6)$alkynyl, phenyl, phenyl-$(C_1-C_4)$alkyl, carboxylic acyl$(C_1-C_6)$, phenyl$(C_1-C_4)$-acyl, nitrile, or $-N(C_1-C_4)_2$alkyl group ;

$R^4$ represents a hydrogen atom, a $(C_1-C_6)$alkyl, $(C_2-C_6)$-alkenyl, $(C_3-C_6)$alkynyl, phenyl or phenyl$(C_1-C_4)$alkyl group, an $-O(C_1-C_4)$alkyl or -Oaryl group, a nitrile group, or an $-NR^5R^6$ group, in which $R^5$ and $R^6$, which may be the same or different, each represents a hydrogen atom, a $(C_1-C_6)$alkyl, $(C_2-C_6)$alkenyl, $(C_3-C_6)$-alkynyl, phenyl or phenyl$(C_1-C_4)$alkyl group, a

$-(CH_2)_m-X-(CH_2)_n-A$ group, or an $-NH_2$ or nitrile group, (m, n, X and A being as defined above), or together with the nitrogen atom to which they are attached may form a 5 or 6 membered ring optionally containing a second nitrogen atom or an oxygen atom, or

$R^3$ and $R^4$ together with the atoms to which they are attached may form a 5 or 6 membered ring, containing one or more further heteroatoms selected from oxygen, nitrogen and sulphur atoms.

2. A compound according to claim 1 wherein

A is selected from the group consisting of thiazolyl, 2-furanyl, phenyl,

X is O or S,

m is 2 or 3,

n is 0 or 1,

p is 1 or 2,

$R^3$ is H, $CH_3$ or $C_2H_5$,

$R^4$ is an amino, $NHR^6$, $C_1$-$C_4$-alkyl or phenoxy group

$R^6$ being a $C_1$-$C_6$alkyl group or a substituted $C_1$-$C_4$alkyl group.

3. A compound according to claim 2 wherein

A is the 2-guanidino-4-thiazolyl group

X is S,

m is 2

n is 1,

p is 1 or 2,

$R^3$ is H, $CH_3$ or $C_2H_5$,

$R^4$ is an $NH_2$, $CH_3$, 4-methylphenoxy, or $NHR^6$ group.

4. A compound according to claim 3, wherein

p is 2,

$R^3$ is $CH_3$

$R^4$ is $NH_2$ or $NHR^6$.

5. A compound according to claim 4, wherein $R^4$ is $NH_2$.

6. A compound according to claim 3, wherein p is 2, $R^3$ is H, $R^4$ is $NH_2$.

7. A compound according to claim 3, wherein p is 2, $R^3$ is $C_2H_5$ and $R^4$ is $NH_2$.

8. A compound according to claim 3 wherein p is 2, $R^3$ is $CH_3$, $R^4$ is $NHCH_2CH_2N(CH_3)_2$.

9. A compound according to claim 3 wherein p is 2, $R^3$ is $CH_3$ and $R^4$ is NH- 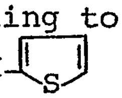

10. A compound according to claim 3, wherein p is 2, $R^3$ is $CH_3$, $R^4$ is NH $CH_2CH_2$- 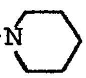

11. A compound according to claim 3, wherein p is 2, $R^3$ is $CH_3$, $R^4$ is NH $CH_2CH_2$- 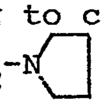

12. A compound according to claim 3 wherein p is 2, $R^3$ is $CH_3$ and $R^4$ is $-NHCH_2CH_2N(C_2H_5)_2$ .

13. A compound according to claim 3, wherein p is 1, $R^3$ is $CH_3$, $R^4$ is $NH_2$.

14. A compound according to claim 3 wherein p is 2, $R^3$ is $CH_3$, $R^4$ is $CH_3$

15. A compound according to claim 2, wherein
A is 3-(1-piperidinylmethyl)-phenyl,
X is O,
m is 3,
n is 0,
p is 2,
$R^3$ is $CH_3$,
$R^4$ is $NH_2$.

0104611
HOE 82/S 016

16. A compound according to claim 2, wherein
A is 3-(1-piperidinyl methyl)phenyl
X is O,
m is 3,
n is 0,
p is 2,
$R^3$ is $CH_3$
$R^4$ is $NHCH_2CH_2N(CH_3)_2$.

17. Process for the production of a compound of the general formula I

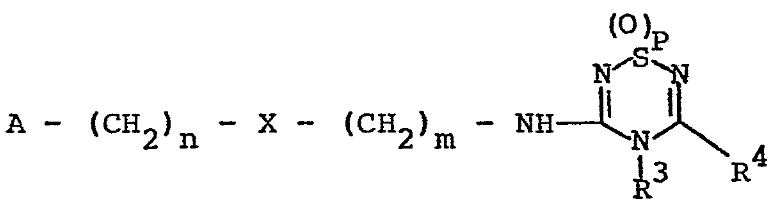

(I)

and of its pharmaceutically acceptable salts in which formula

A represents a phenyl, imidazolyl, thiazolyl, furyl, thienyl, or pyridyl radical; which radical may contain one or two substituents, the first being selected from lower alkyl groups, and the second from lower alkyl, guanidino, and $-CH_2NR^1R^2$ groups, $R^1$ and $R^2$, which may be the same or different, each representing a hydrogen atom or a $(C_1-C_6)$alkyl group, or together with the nitrogen atom to which they are attached, may form a pyrrolidine, piperidine, morpholine, or N-methylpiperazine ring;

X represents -O-, -S-, or $-CH_2$;

n represents 0 or 1;

m represents 2 or 3;

p represents 1 or 2;

$R^3$ represents a hydrogen atom or a $(C_1-C_6)$alkyl, $(C_2-C_6)$-alkenyl, $(C_3-C_6)$alkynyl, phenyl, phenyl(lower)alkyl, carboxylic acyl$(C_1-C_6)$, phenyl(lower)acyl, nitrile, or $-N(alkyl)_2$ group;

$R^4$ represents a hydrogen atom, a $(C_1-C_6)$alkyl, $(C_2-C_6)$-alkenyl, $(C_3-C_6)$alkynyl, phenyl or phenyl(lower)alkyl group, an -O(lower)alkyl or -Oaryl group, a nitrile group, or an $-NR^5R^6$ group, in which $R^5$ and $R^6$, which may be the same or different, each represents a hydrogen atom, a $(C_1-C_6)$alkyl, $(C_2-C_6)$alkenyl, $(C_3-C_6)$alkynyl, phenyl or phenyl(lower)alkyl group, a $-(CH_2)_m-X-(CH_2)_n-A$ group, or an $-NH_2$ or nitrile group, (m, n, X and A being as defined above), or together with the nitrogen atom to which they are attached may form a 5 or 6 membered ring optionally containing a second nitrogen atom or an oxygen atom, or

$R^3$ and $R^4$ together with the atoms to which they are attached may form a 5 or 6 membered ring, containing one or more further heteroatoms selected from oxygen, nitrogen and sulphur atoms

which process comprises

a) reacting a 1,2,4,6-thiatriazine of the general formula II

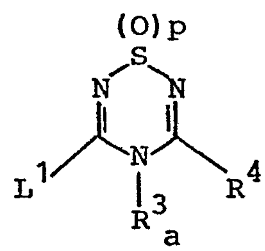

II

in which $L^1$ represents a halogen atom, an alkoxy or aryloxy group, an alkylthio or arylthio group, or an alkylsulphonyl or arylsulphonyl group, p and $R^4$ are as defined above, and $R^3_a$ is as defined above for $R^3$ except that $R^3_a$ may not represent a hydrogen atom, with a compound of the general formula III

$$A - (CH_2)_n - X - (CH_2)_m - NH_2 \qquad III$$

in which A, X, m and n are as defined for formula I, or

b) reacting a 1,2,4,6-thiatriazine of the general formula IV

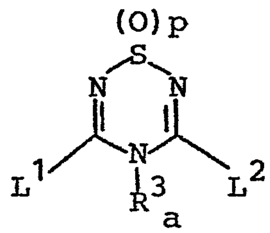

IV

in which $L^1$ and $L^2$, which may be the same or different, each represents a halogen atom, an alkoxy or aryloxy group, an alkylthio or arylthio group, or an alkylsulphonyl or arylsulphonyl group, and p and $R^3_a$ are as defined above, with a compound of formula III as defined above, and reacting the resulting compound of the formula V

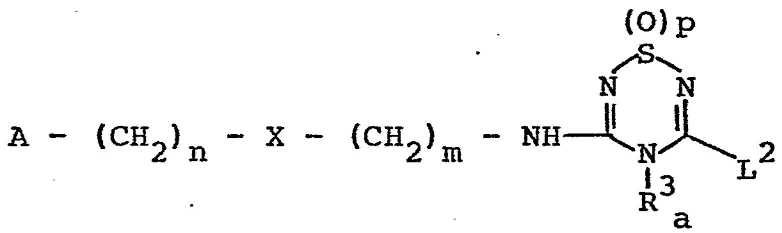

V

with an amino compound $HNR^5_aR^6_a$ in which $R^5_a$ and $R^6_a$ are as defined for $R^5$ and $R^6$ in formula I and, in addition, either or both of $R^5_a$ and $R^6_a$ may represent a trialkylsilyl group, for example, a $-(CH_3)_3Si$ group; or

c) reacting a 1,2,4,6-thiatriazine of the general formula II as defined above with an amino thiol of formula VI

$$HX_a - (CH_2)_m - NH_2 \qquad VI$$

in which $X_a$ is -O- or -S-, and m is as defined for formula I, and reacting the resulting compound of formula VII

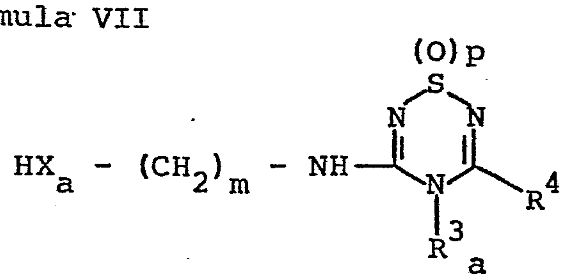

VII

in which $R^3_a$, $R^4$, p and m are as defined above, with a compound of the general formula VIII

$$A - (CH_2)_{n^1} - L^3 \qquad VIII$$

in which A is as defined for formula I, $n^1$ represents 1, and $L^3$ is a suitable leaving group, for example, a halogen atom, a hydroxy group, an alkoxy group, or a sulphonate ester, and, if desired, carrying out any one or more of the following reactings in any desired order:

(i) converting a group $R^3_a$ into a hydrogen atom,

(ii) converting a group $R^3_a$ and/or a group $R^4$ into another group $R^3$ and/or $R^4$, respectively,

(iii) converting an acid addition salt of formula I into the corresponding free base or converting a free base into an acid addition salt,

(iv) oxidising a compound or formula I in which p represents 1 to give the corresponding compound in which p represents 2.

18. A pharmaceutical preparation consisting of a compound of general formula I as claimed in claim 1 and of a pharmaceutically suitable carrier.

19. Method of treating human beings by administering from 0,1 to 10 mg of a compound of formula I as claimed in claim 1 per kilogramm body weight in form of a suitable pharmaceutical preparation.

Claims for Austria:

1. Process for the production of a compound of the general formula I

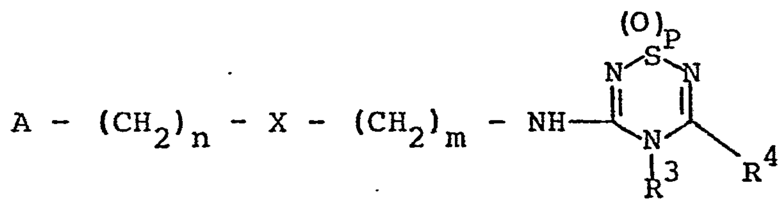

(I)

and of its pharmaceutically acceptable salts in which formula

A represents a phenyl, imidazolyl, thiazolyl, furyl, thienyl, or pyridyl radical; which radical may contain one or two substituents, the first being selected from lower alkyl groups, and the second from lower alkyl, guanidino, and $-CH_2NR^1R^2$ groups, $R^1$ and $R^2$, which may be the same or different, each representing a hydrogen atom or a $(C_1-C_6)$alkyl group, or together with the nitrogen atom to which they are attached, may form a pyrrolidine, piperidine, morpholine, or N-methylpiperazine ring;

X represents -O-, -S-, or $-CH_2$;

n represents 0 or 1;

m represents 2 or 3;

p represents 1 or 2;

$R^3$ represents a hydrogen atom or a $(C_1-C_6)$alkyl, $(C_2-C_6)$-alkenyl, $(C_3-C_6)$alkynyl, phenyl, phenyl(lower)alkyl, carboxylic acyl$(C_1-C_6)$, phenyl(lower)acyl, nitrile, or $-N(alkyl)_2$ group;

$R^4$ represents a hydrogen atom, a $(C_1-C_6)$alkyl, $(C_2-C_6)$-alkenyl, $(C_3-C_6)$alkynyl, phenyl or phenyl(lower)alkyl group, an -O(lower)alkyl or -Oaryl group, a nitrile group, or an $-NR^5R^6$ group, in which $R^5$ and $R^6$, which may be the same or different, each represents a hydrogen atom, a $(C_1-C_6)$alkyl, $(C_2-C_6)$alkenyl, $(C_3-C_6)$alkynyl, phenyl or phenyl(lower)alkyl group, a $-(CH_2)_m-X-(CH_2)_n-A$ group, or an $-NH_2$ or nitrile group, (m, n, X and A being as defined above), or together with the nitrogen atom to which they are attached may form a 5 or 6 membered ring optionally containing a second nitrogen atom or an oxygen atom, or

$R^3$ and $R^4$ together with the atoms to which they are attached may form a 5 or 6 membered ring, containing one or more further heteroatoms selected from oxygen, nitrogen and sulphur atoms

which process comprises

a) reacting a 1,2,4,6-thiatriazine of the general formula II

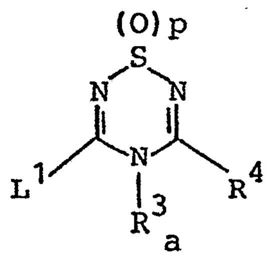

II

in which $L^1$ represents a halogen atom, an alkoxy or aryloxy group, an alkylthio or arylthio group, or an alkylsulphonyl or arylsulphonyl group, p and $R^4$ are as defined above, and $R^3_a$ is as defined above for $R^3$ except that $R^3_a$ may not represent a hydrogen atom, with a compound of the general formula III

$$A - (CH_2)_n - X - (CH_2)_m - NH_2 \qquad III$$

in which A, X, m and n are as defined for formula I, or

b) reacting a 1,2,4,6-thiatriazine of the general formula IV

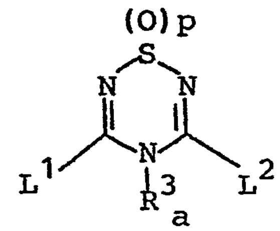

IV

in which $L^1$ and $L^2$, which may be the same or different, each represents a halogen atom, an alkoxy or aryloxy group, an alkylthio or arylthio group, or an alkylsulphonyl or arylsulphonyl group, and p and $R^3_a$ are as defined above, with a compound of formula III as defined above, and reacting the resulting compound of the formula V

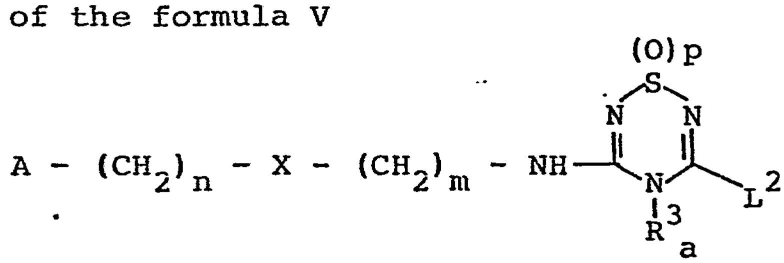

V

with an amino compound $HNR^5_aR^6_a$ in which $R^5_a$ and $R^6_a$ are as defined for $R^5$ and $R^6$ in formula I and, in addition, either or both of $R^5_a$ and $R^6_a$ may represent a trialkylsilyl group, for example, a $-(CH_3)_3Si$ group; or

c) reacting a 1,2,4,6-thiatriazine of the general formula II as defined above with an amino thiol of formula VI

$$HX_a - (CH_2)_m - NH_2 \qquad VI$$

in which $X_a$ is -O- or -S-, and m is as defined for formula I, and reacting the resulting compound of formula VII

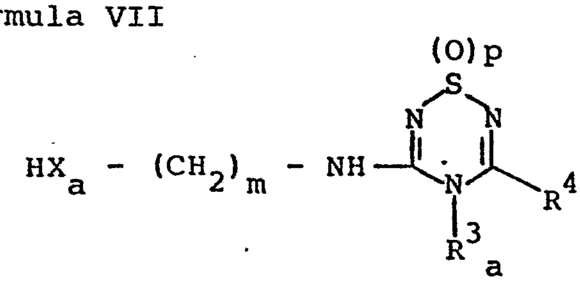

VII

in which $R^3_a$, $R^4$, p and m are as defined above, with a compound of the general formula VIII

$$A - (CH_2)_{n^1} - L^3 \qquad VIII$$

in which A is as defined for formula I, $n^1$ represents 1, and $L^3$ is a suitable leaving group, for example, a halogen atom, a hydroxy group, an alkoxy group, or a sulphonate ester, and, if desired, carrying out any one or more of the following reactings in any desired order:

(i) converting a group $R^3_a$ into a hydrogen atom,

(ii) converting a group $R^3$ and/or a group $R^4$ into another group $R^3$ and/or $R^4$, respectively,

(iii) converting an acid addition salt of formula I into the corresponding free base or converting a free base into an acid addition salt,

(iv) oxidising a compound or formula I in which p represents 1 to give the corresponding compound in which p represents 2.

2. A process according to claim 1 wherein

A is selected from the group consisting of thiazolyl, 2-furanyl, phenyl,

X is O or S,

m is 2 or 3,

n is 0 or 1,

p is 1 or 2,

$R^3$ is H, $CH_3$ or $C_2H_5$,

$R^4$ is an amino, $NHR^6$, $C_1$-$C_4$-alkyl or phenoxy group

$R^6$ being a $C_1$-$C_6$alkyl group or a substituted $C_1$-$C_4$alkyl group.

3. A process according to claim 2 wherein
A is the 2-guanidino-4-thiazolyl group
X is S,
m is 2
n is 1,
p is 1 or 2,
$R^3$ is H, $CH_3$ or $C_2H_5$,
$R^4$ is an $NH_2$, $CH_3$, 4-methylphenoxy, or $NHR^6$ group.

4. A process according to claim 3, wherein
p is 2,
$R^3$ is $CH_3$
$R^4$ is $NH_2$ or $NHR^6$.

5. A process according to claim 4, wherein
$R^4$ is $NH_2$.

6. A process according to claim 3, wherein p is 2, $R^3$ is H, $R^4$ is $NH_2$.

7. A process according to claim 3, wherein p is 2, $R^3$ is $C_2H_5$ and $R^4$ is $NH_2$.

8. A process according to claim 3 wherein p is 2, $R^3$ is $CH_3$, $R^4$ is $NHCH_2CH_2N(CH_3)_2$.

9. A process according to claim 3 wherein p is 2, $R^3$ is $CH_3$ and $R^4$ is NH- 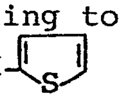

10. A process according to claim 3, wherein p is 2, $R^3$ is $CH_3$, $R^4$ is NH $CH_2CH_2$- 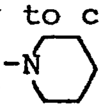

11. A process according to claim 3, wherein p is 2, $R^3$ is $CH_3$, $R^4$ is NH $CH_2CH_2$- 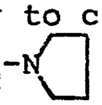

12. A process according to claim 3 wherein p is 2, $R^3$ is $CH_3$ and $R^4$ is $-NHCH_2CH_2N(C_2H_5)_2$ .

13. A process according to claim 3, wherein p is 1, $R^3$ is $CH_3$, $R^4$ is $NH_2$.

14. A process according to claim 3 wherein p is 2, $R^3$ is $CH_3$, $R^4$ is $CH_3$

15. A process according to claim 2, wherein
A is 3-(1-piperidinylmethyl)-phenyl,
X is O,
m is 3,
n is 0,
p is 2,
$R^3$ is $CH_3$,
$R^4$ is $NH_2$.

16. A process according to claim 2, wherein
A is 3-(1-piperidinyl methyl)phenyl
X is 0,
m is 3,
n is 0,
p is 2,
$R^3$ is $CH_3$
$R^4$ is $NHCH_2CH_2N(CH_3)_2$.

17. A pharmaceutical preparation consisting of a compound of general formula I produced according to claim 1 and of a pharmaceutically suitable carrier.

18. Method of treating human beings by administering from 0,1 to 10 mg of a compound of formula I as claimed in claim 1 per kilogramm body weight in form of a suitable pharmaceutical preparation.

BAD ORIGINAL